# EUROPEAN PATENT SPECIFICATION

(11) **EP 1 912 708 B1**
(45) Date of publication and mention of the grant of the patent: **08.09.2010**
(21) Application number: 06768380.5
(22) Date of filing: 24.07.2006
(51) Int. Cl.: A61Q 19/00, A61K 8/67, A61K 8/37, A61K 8/39, A61K 8/73, A61K 31/355, A61K 47/14, A61K 47/26

(54) **EMULSIFIED SKIN EXTERNAL PREPARATION AND METHOD FOR STABILIZING THE SKIN EXTERNAL PREPARATION**
EMULGIERTES TOPISCHES HAUTPRÄPARAT UND VERFAHREN ZUR STABILISIERUNG DES TOPISCHEN HAUTPRÄPARATS
PRÉPARATION ÉMULSIFIÉE À USAGE EXTERNE POUR LA PEAU ET PROCÉDÉ SERVANT À STABILISER LA PRÉPARATION À USAGE EXTERNE POUR LA PEAU

(30) Priority: 27.07.2005 JP 2005217648; 05.10.2005 JP 2005292351
(43) Date of publication of application: 23.04.2008
(73) Proprietor: Showa Denko K.K., Tokyo 105-8518 (JP)
(72) Inventor: YONEDA, Tadashi, Chiba-shi, Chiba 267-0056 (JP); ITO, Naoko, Chiba-shi, Chiba 267-0056 (JP); FURUYA, Kazuo, Chiba-shi, Chiba 267-0056 (JP)
(74) Representative: Strehl Schübel-Hopf & Partner
(86) International application number: PCT/JP2006/315071
(87) International publication number: WO 2007/013634

(56) References cited:
- WO-A-2005/102267
- US-A- 2 988 553
- US-A1- 2005 048 011

## Description

### TECHNICAL FIELD

The present invention relates to a stable emulsifiedskin external preparation containing a tocopherol glycinate derivative.

The invention also relates to a method for stabilizing an emulsified skin external preparation containing a tocopherol glycinate derivative.

### BACKGROUND ART

Tocopherol (α-tocopherol, β-tocopherol, γ-tocopherol and δ-tocopherol) known as vitamin E and derivatives thereof such as tocopherol acetate and tocopherol nicotinate (hereinafter, the tocopherols) are known to produce effects such as antioxidation, biomembrane stabilization, immunostimulation and facilitation of blood circulation. They have been used in medicines, cosmetics and diets.

The present applicant has reported skin external preparations containing tocopherol glycinate and derivatives thereof based on the finding that among the tocopherols, the tocopherol glycinate and derivatives thereof have particularly excellent conversion efficiency into active tocopherol in skin tissues (JP-A-2004-2277 and JP-A-2004-2278).

However, formulating the tocopherol glycinate derivatives into emulsified skin external preparations has often resulted in changes in appearance such as viscosity reduction and separation during storage.

### DISCLOSURE OF INVENTION

It is an object of the invention to provide an emulsified skin external preparation containing a tocopherol glycinate derivative that has excellent storage stability without viscosity reduction during storage.

It is another object of the invention that the provision of the skin external preparation provides a method for stabilizing the skin external preparation containing a tocopherol glycinate derivative.

The present inventors studied diligently in view of the aforesaid problems and have found that the problems are solved by adding specific fatty acid esters. The invention has been completed based on the finding.

The invention is concerned with the following.
[1] An emulsified skin external preparation comprising 0.03 to 25% by mass of a tocopherol glycinate derivative, and 0.05 to 25% by mass of at least one fatty acid ester selected from the group consisting of glycerin mono fatty acid ester, polyglycerin fatty acid ester and dextrin fatty acid ester, the tocopherol glycinate derivative being represented by Formula (1): 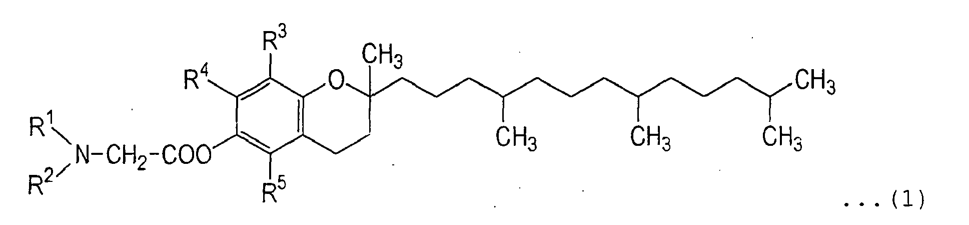 wherein R¹ and R² are each a lower alkyl group or a hydrogen atom and cannot be hydrogen atoms at the same time, and R³, R⁴ and R⁵ are each a hydrogen atom or a methyl group,
   0.20.wt% of a tocopherol glycine ester, 3.00 wt% of a ascorbic acid-2-phosphoric acid ester, 73.10 wt% of purified water, 0.01 wt% of surfactin Na; or
   1.00 wt% of a tocopherol glycine ester, 1.00 wt% of a ascorbic acid-2-phosphoric acid ester, 74.30 wt% of purified water, 0.01 wt% of surfactin Na,
   wherein the tocopherol glycine ester is selected from the group consisting of dl-α-tocopherol dimethylglycine ester hydrochloride, d-α-tocopherol dimethylglycine ester hydrochloride, d-δ-tocopherol dimethylglycine ester hydrochloride, d-γ-tocopherol dimethylglycine ester hydrochloride, dl-α-tocopherol sarcosine ester hydrochloride, d-α-tocopherol sarcosine ester hydrochloride, d-δ-tocopherol sarcosine ester hydrochloride, and d-γ-tocopherol sarcosine ester hydrochloride,
   and wherein the ascorbic acid-2-phosphoric acid ester is selected from the group consisting of ascorbic acid-2-phosphoric acid ester sodium salt, ascorbic acid-2-phosphoric acid ester magnesium salt, ascorbic acid-2-phosphoric acid ester-6-palmitic acid ester sodium salt and ascorbic acid-2-phosphoric acid ester-6-hexyldecanoic acid ester sodium salt.
[2] The emulsified skin external preparation as described in [1], wherein the tocopherol glycinate derivative is tocopherol N,N-dimethylglycinate or tocopherol sarcosinate.
[3] The emulsified skin external preparation as described in [1] or [2], wherein the tocopherol glycinate derivative is at least one derivative selected from the group consisting of α-tocopherol glycinate derivatives, γ-tocopherol glycinate derivatives and δ-tocopherol glycinate derivatives.
[4] The emulsified skin external preparation as described in any one of [1] to [3], wherein the tocopherol glycinate derivative is at least one derivative selected from the group consisting of dl-α-tocopherol glycinate derivatives, d-α-tocopherol glycinate derivatives, d-γ-tocopherol glycinate derivatives and d-δ-tocopherol glycinate derivatives.
[5] The emulsified skin external preparation as described in [1], wherein the glycerin mono fatty acid ester is at least one fatty acid ester selected from the group consisting of glyceryl laurate, glyceryl myristate, glyceryl stearate, glyceryl isostearate, glyceryl behenate, glyceryl linoleate, glyceryl oleate, glyceryl cocofatty acid ester, glyceryl ricinoleate, glyceryl hydroxystearate and glyceryl erucate.
[6] The emulsified skin external preparation as described in [1], wherein the polyglycerin fatty acid ester is a fatty acid ester of polyglycerin having two to ten glycerin molecules.
[7] The emulsified skin external preparation as described in [6], wherein the fatty acid ester of polyglycerin having two to ten glycerin molecules is at least one fatty acid ester selected from the group consisting of poly (2) glyceryl monolaurate, poly (2) glyceryl monomyristate, poly (2) glyceryl monostearate, poly (2) glyceryl monoisostearate, poly (10) glyceryl monolaurate, poly (10) glyceryl monomyristate, poly (10) glyceryl monostearate and poly (10) glyceryl monoisostearate.
[8] The emulsified skin external preparation as described in [1], wherein the dextrin fatty acid ester is at least one fatty acid ester selected from the group consisting of dextrin myristate, dextrin palmitate, dextrin (palmitate/octanoate) and dextrin stearate.
[9] An emulsified cosmetic comprising the emulsified skin external preparation of any one of [1] to [8].
[10] A method for stabilizing an emulsified skin external preparation, **characterized in that** a tocopherol glycinate derivative is used in combination with at least one fatty acid ester selected from the group consisting of glycerin mono fatty acid ester, polyglycerin fatty acid ester and dextrin fatty acid ester, the tocopherol glycinate derivative being represented by Formula (1): 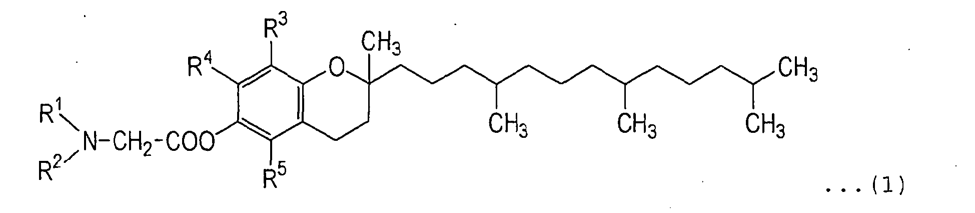 wherein R¹ and R² are each a lower alkyl group or a hydrogen atom and cannot be hydrogen atoms at the same time, and R³, R⁴ and R⁵ are each a hydrogen atom or a methyl group, with the proviso that the claimed method does not apply to the preparations excluded by the disclaimer of claim 1.

The emulsified skin external preparation according to the present invention has excellent storage stability such that defects such as viscosity reduction and separation during storage are prevented. The emulsified skin external preparation is useful as general skin external preparations, particularly useful as cosmetics.

### BEST MODE FOR CARRYING OUT THE INVENTION

The emulsified skin external preparation, emulsified cosmetic, and method for stabilizing the emulsified skin external preparation according to the present invention will be described in detail below.

### <Emulsified skin external preparation>

The emulsified skin external preparation includes a tocopherol glycinate derivative, and at least one fatty acid ester selected from the group consisting of glycerin mono fatty acid ester, polyglycerin fatty acid ester and dextrin fatty acid ester.

### <Tocopherol glycinate derivative>

The tocopherol glycinate derivative used in the invention is represented by Formula (1): 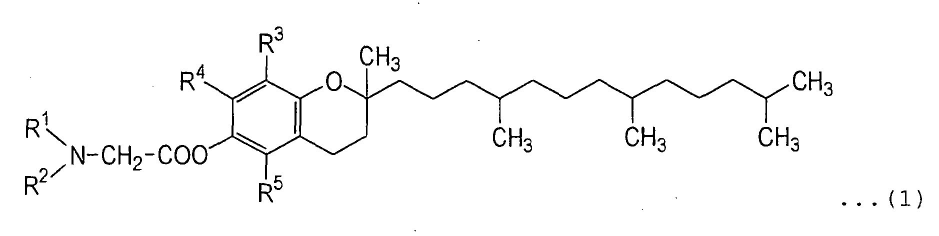 wherein R¹ and R² are each a lower alkyl group or a hydrogen atom and cannot be hydrogen atoms at the same time, and R³, R⁴ and R⁵ are each a hydrogen atom or a methyl group.

As shown in Formula (1), the tocopherol glycinate derivative is composed of a tocopherol skeleton and a glycine derivative skeleton.

Examples of the tocopherols for the tocopherol skeleton include α-tocopherol, β-tocopherol, γ-tocopherol and δ-tocopherol, with α-tocopherol, γ-tocopherol and δ-tocopherol being preferred.

The tocopherols have an asymmetric carbon at the 2-position of the chromanol ring, and stereoisomers such as d-isomer and dl-isomer are possible. The present invention may employ any of such isomers.

Preferred examples of the tocopherols for the tocopherol skeleton include dl-α-tocopherol, d-α-tocopherol, d-γ-tocopherol and d-δ-tocopherol, with dl-α-tocopherol, d-α-tocopherol and d-γ-tocopherol being more preferable.

With regard to the glycine derivative skeleton, the lower alkyl groups R¹ and R² in Formula (1) include linear or branched alkyl groups of 1 to 6 carbon atoms. Specific examples include methyl, ethyl, n-propyl, n-butyl, isopropyl, isobutyl, 1-methylpropyl, tert-butyl, n-pentyl, 1-ethylpropyl, isoamyl and n-hexyl groups, with methyl group being particularly preferred.

That is, N,N-dimethylglycine and sarcosine are preferable glycine derivatives for the tocopherol glycinate derivatives.

In the skin external preparation, the tocopherol glycinate derivatives may be used singly or in combination of two or more kinds.

The tocopherol glycinate derivatives generally account for 0.03 to 25% by mass, preferably 0.1 to 10% by mass, more preferably 0.5 to 5% by mass of the emulsified skin external preparation. When the amount of the tocopherol glycinate derivatives is less than the above range, the skin external preparation often does not produce desired effects. Any amount exceeding the above range is not effective because further improvements in effects are not expected.

### <Glycerin mono fatty acid ester>

The glycerin mono fatty acid ester used in the invention is a compound in which one molecule of a fatty acid is ester bonded to any hydroxyl group of glycerin. The ester is otherwise called monoacylglycerol or monoglyceride. Examples of the fatty acids include straight-chain fatty acids, branched-chain fatty acids, saturated fatty acids, unsaturated fatty acids and hydroxy fatty acids.

Examples of the glycerin mono fatty acid esters include glyceryl laurate, glyceryl myristate, glyceryl stearate, glyceryl linoleate, glyceryl oleate, glyceryl isostearate, glyceryl behenate, glyceryl erucate, glyceryl cocofatty acid ester, glyceryl ricinoleate, glyceryl hydroxystearate, wheat germ oil fatty acid monoglyceride, safflower oil fatty acid monoglyceride, hydrogenated soybean fatty acid monoglyceride, saturated fatty acid monoglyceride, cotton seed oil fatty acid monoglyceride, tallow fatty acid monoglyceride and lanolin fatty acid monoglyceride.

Of these, glyceryl laurate, glyceryl myristate, glyceryl stearate, glyceryl linoleate, glyceryl oleate, glyceryl isostearate, glyceryl behenate, glyceryl erucate, glyceryl cocofatty acid ester, glyceryl ricinoleate and glyceryl hydroxystearate are preferable, and glyceryl myristate, glyceryl stearate and glyceryl isostearate are more preferable.

The glycerin mono fatty acid esters may be used singly or in combination of two or more kinds.

### <Polyglycerin fatty acid ester>

The polyglycerin fatty acid ester used in the invention is a compound that results when glycerin is dehydrated and condensed into a polyglycerin, and one or more molecules of a fatty acid are ester bonded to any hydroxyl group in the polyglycerin. Preferred examples of the polyglycerin fatty acid esters include compounds in which one or more molecules of a fatty acid are ester bonded to any hydroxyl group in the polyglycerins having an average polymerization degree of 2 to 10. Examples of the fatty acids include straight-chain fatty acids, branched-chain fatty acids, saturated fatty acids, unsaturated fatty acids and hydroxy fatty acids.

Examples of the polyglycerin fatty acid esters include diglyceryl isopalmitate, poly (4-10) glyceryl monolaurate, poly (10) glyceryl monomyristate, poly (2-10) glyceryl monostearate, poly (2-10) glyceryl monoisostearate, poly (2-10) glyceryl monooleate, diglyceryl sesquioleate, poly (2-10) glyceryl diisostearate, poly (6-10) glyceryl distearate, diglyceryl triisostearate, poly (10) glyceryl tristearate, poly (10) glyceryl trioleate, poly (2) glyceryl tetraisostearate, decaglyceryl pentastearate, poly (6-10) glyceryl pentaoleate, poly (10) glyceryl heptastearate, decaglyceryl decastearate, poly (10) glyceryl decaoleate and condensed poly (6) glyceryl ricinoleate.

Of these, poly (2) glyceryl monolaurate, poly (2) glyceryl monomyristate, poly (2) glyceryl monostearate, poly (2) glyceryl monoisostearate, poly (10) glyceryl monolaurate, poly (10) glyceryl monomyristate, poly (10) glyceryl monostearate and poly (10) glyceryl monoisostearate are preferable, and poly (10) glyceryl monomyristate, poly (10) glyceryl monostearate and poly (10) glyceryl monoisostearate are more preferable.

The polyglycerin fatty acid esters may be used singly or in combination of two or more kinds.

### <Dextrin fatty acid ester>

The dextrin fatty acid ester used in the invention is a higher fatty acid ester of starch-derived dextrin. Examples of the fatty acids include straight-chain fatty acids, branched-chain fatty acids, saturated fatty acids and unsaturated fatty acids.

Examples of the dextrin fatty acid esters include dextrin myristate, dextrin palmitate, dextrin (palmitate/octanoate) and dextrin stearate.

Of these, dextrin myristate and dextrin palmitate are preferable, and dextrin palmitate is particularly preferable.

The dextrin fatty acid esters may be used singly or in combination of two or more kinds.

The fatty acid esters generally account for 0.05 to 25% by mass, preferably 1.5 to 10% by mass, more preferably 2 to 5% by mass of the emulsified skin external preparation. This amount of the fatty acid esters enables the stable skin external preparation.

### <Other components>

The skin external preparation may contain a component that is commonly used in the skin external preparations, together with the tocopherol glycinate derivative and at least one fatty acid ester selected from the glycerin mono fatty acid esters, polyglycerin fatty acid esters and dextrin fatty acid esters. Examples of such additional components include:
hydrocarbons such as ozokerite, α-olefin oligomers, light isoparaffin, light liquid isoparaffin, squalene, Squalane, synthetic squalane, vegetable squalane, ceresin, paraffin, polyethylene powder, polybutene, microcrystalline wax, liquid isoparaffin, liquid paraffin, mineral oil and vaseline;
natural fats and oils, such as natural waxes including jojoba oil, carnauba wax, candelilla wax, rice bran wax, shellac, lanolin, mink oil wax, whale wax, sugarcane wax, sperm oil, beeswax and montan wax; avocado oil, almond oil, olive oil, extra virgin olive oil, sesame oil, rice bran oil, rice oil, rice germ oil, corn oil, safflower oil, soybean oil, maize oil, rapeseed oil, persic oil, palm kernel oil, palm oil, castor oil, sunflower oil, high oleic sunflower oil, grape seed oil, cotton seed oil, coconut oil, hydrogenated coconut oil, beef tallow, hydrogenated oil, horse oil, mink oil, egg yolk oil, egg yolk fatty oil, rose hip oil, kukui nut oil, evening primrose oil, wheat germ oil, peanut oil, camellia oil, sasanqua oil, cacao butter, Japanese wax, beef bone fat, neatsfoot oil, lard, horse fat, mutton tallow, shea butter, macadamia nut oil and meadowfoam oil;
fatty acids such as lauric acid, myristic acid, palmitic acid, stearic acid, behenic acid, oleic acid, linolic acid, linolenic acid, γ-linolenic acid, isostearic acid, 12-hydroxystearic acid, undecylenic acid and coconut fatty acid;
higher alcohols such as isostearyl alcohol, octyldodecanol, hexyldecanol, cholesterol, phytosterol, lauryl alcohol, myristyl alcohol, cetanol, stearyl alcohol, oleyl alcohol, behenyl alcohol and cetostearyl alcohol;
alkyl glyceryl ethers such as batyl alcohol, chimyl alcohol, selachyl alcohol and isostearyl glyceryl ether;
esters such as isopropyl myristate, butyl myristate, isopropyl palmitate, ethyl stearate, butyl stearate, ethyl oleate, ethyl linoleate, isopropyl linoleate, cetyl caprylate, hexyl laurate, isooctyl myristate, decyl myristate, myristyl myristate, cetyl myristate, octadecyl myristate, cetyl palmitate, stearyl stearate, decyl oleate, oleyl oleate, cetyl ricinoleate, isostearyl laurate, isotridecyl myristate, isocetyl myristate, isostearyl myristate, octyldodecyl myristate, 2-ethylhexyl palmitate, isocetyl palmitate, isostearyl palmitate, 2-ethylhexyl stearate, isocetyl stearate, isodecyl oleate, octyldodecyl oleate, octyldodecyl ricinoleate,
ethyl isostearate, isopropyl isostearate, cetyl 2-ethylhexanoate, cetostearyl 2-ethylhexanoate, stearyl 2-ethylhexanoate, hexyl isostearate, ethylene glycol dioctanoate, ethylene glycol dioleate, propylene glycol dicaprylate, propylene glycol di(caprylate caprate), propylene glycol dicaprate, propylene glycol dioleate, neopentyl glycol dicaprate, neopentyl glycol dioctanoate, glyceryl tricaprylate, glyceryl tri-2-ethylhexanoate, glyceryl tri(caprylate caprate), glyceryl tri(caprylate caprate stearate), glyceryl triundecylate, glyceryl triisopalmitate, glyceryltriisostearate, trimethylolpropane tri-2-ethylhexanoate, trimethylolpropane triisostearate,
pentaerythrityl tetra-2-ethylhexanoate, pentaerythrityl tetramyristate, pentaerythrityl tetraisostearate, diglyceryl tetraisostearate, octyldodecyl neopentanoate, isocetyl octanoate, isostearyl octanoate, 2-ethylhexyl isopelargonate, hexyldecyl dimethyloctanoate, octyldodecyl dimethyloctanoate, 2-ethylhexyl isopalmitate, isocetyl isostearate, isostearyl isostearate, octyldodecyl isostearate, lauryl lactate, myristyl lactate, cetyl lactate, octyldodecyl lactate, triethyl citrate, acetyltriethyl citrate, acetyltributyl citrate, trioctyl citrate, triisocetyl citrate, trioctyldodecyl citrate, diisostearyl malate, 2-ethylhexyl hydroxystearate, di-2-ethylhexyl succinate,
diisopropyl adipate, diisobutyl adipate, dioctyl adipate, diheptylundecyl adipate, diethyl sebacate, diisopropyl sebacate, dioctyl sebacate, cholesteryl stearate, cholesteryl isostearate, cholesteryl hydroxystearate, cholesteryl oleate, dihydrocholesteryl oleate, phytosteryl isostearate, phytosteryl oleate, isocetyl 12-stearoylhydroxystearate, stearyl 12-stearoylhydroxystearate, isostearyl 12-stearoylhydroxystearate, polyoxyethylene (3) polyoxypropylene (1) cetyl ether acetate, polyoxyethylene (3) polyoxypropylene (1) isocetyl ether acetate, isononyl isononanoate, octyl isononanoate, tridecyl isononanoate and isotridecyl isononanoate;
silicone oils such as methyl polysiloxane, methylphenyl polysiloxane, methylhydrogen polysiloxane, methyl cyclopolysiloxane, octamethyl cyclotetrasiloxane, decamethyl cyclopentasiloxane, dodecamethyl cyclohexasiloxane, octamethyl trisiloxane, decamethyl tetrasiloxane, tetradecamethyl hexasiloxane, highly polymerized methyl polysiloxane, dimethyl siloxane/methyl(polyoxyethylene)siloxane/methyl(polyoxypropylene)siloxane copolymer, dimethyl siloxane/methyl(polyoxyethylene)siloxane copolymer, dimethyl siloxane/methyl(polyoxypropylene)siloxane copolymer, dimethyl siloxane/methyl cetyloxysiloxane copolymer, dimethyl siloxane/methyl stearoxysiloxane copolymer, polyether-modified silicones, alcohol-modified silicones, alkyl-modified silicones and amino-modified silicones;
polymers such as sodium alginate, carrageenan, agar, furcelleran, cyamoposis gum, pyrus cydonia seed, konj ac mannan, tamarind gum, tara gum, dextrin, starch, locust bean gum, gum arabic, ghatti gum, karaya gum, tragacanth gum, arabinogalactan, pectin, marmelo, chitosan, starch, curdlan, xanthan gum, gellan gum, cyclodextrin, dextran, pullulan, microcrystalline cellulose, methylcellulose, ethylcellulose, hydroxyethylcellulose, hydroxypropylcellulose, hydroxypropylmethylcellulose, carboxymethylcellulose, carboxy starch, cationized cellulose, starch phosphate, cationized cyamoposis gum, carboxymethyl/hydroxypropylated cyamoposis gum, hydroxypropylated cyamoposis gum, albumin, casein, gelatin,
sodium polyacrylate, polyacrylic acid amide, carboxyvinyl polymers, polyethyleneimine, highly polymerized polyethylene glycol, polyvinyl alcohol, polyvinylpyrrolidone, polyvinyl ether, polyacrylamide, acrylic acid copolymers, methacrylic acid copolymers, maleic acid copolymers, vinylpyridine copolymers, ethylene/acrylic acid copolymers, vinylpyrrolidone polymers, vinyl alcohol/vinylpyrrolidone copolymers, nitrogen-substituted acrylamide polymers, amino-modified silicones, cationized polymers, dimethylacryl ammonium polymers, acrylic acid-based anionic polymers, methacrylic acid-based anionic polymers, modified silicones, alkyl(C₁₀₋₃₀) acrylate or methacrylate copolymers and polyoxyethylene/polyoxypropylene copolymer;
alcohols such as ethanol, isopropyl alcohol, 1-butanol, 2-butanol and benzyl alcohol;
dihydric alcohols such as ethylene glycol, propylene glycol, 1,3-butanediol, 3-methyl-1,3-butanediol, 1,2-pentanediol, 1,2-hexanediol, diethylene glycol, triethylene glycol, polyethylene glycol, dipropylene glycol and polypropylene glycol;
trihydric alcohols such as glycerol;
ethers of trihydric alcohols such as diglycerol and polyglycerol;
sugar alcohols such as mannitol, sorbitol, xylitol, maltitol, erythritol and pentaerythritol;
monosaccharides such as glucose, fructose and xylose;
oligosaccharides such as sucrose, lactose, maltose and trehalose;
anionic surfactants such as potassium coconut fatty acid ester, sodium coconut fatty acid ester, triethanolamine coconut fatty acid ester, potassium laurate, sodium laurate, triethanolamine laurate, potassium myristate, sodium myristate, isopropanolamine myristate, potassium palmitate, sodium palmitate, isopropanolamine palmitate, potassium stearate, sodium stearate, triethanolamine stearate, potassium oleate, sodium oleate, sodium castor oil fatty acid ester, zinc undecylenate, zinc laurate, zinc myristate, magnesium myristate, zinc palmitate, zinc stearate, calcium stearate, magnesium stearate, aluminum stearate, calcium myristate, magnesium myristate, aluminum dimyristate, aluminum isostearate,
polyoxyethylene laurylether acetic acid, sodium polyoxyethylene laurylether acetate, polyoxyethylene tridecylether acetic acid, sodium polyoxyethylene tridecylether acetate, sodium stearoyl lactate, sodium isostearoyl lactate, lauroylsarcosine sodium, sarcosine coconut fatty acid ester, sarcosine sodium coconut fatty acid ester, sarcosine triethanolamine coconut fatty acid ester, lauroyl sarcosine, lauroyl sarcosine potassium, lauroyl sarcosine triethanolamine, oleoyl sarcosine, myristoyl sarcosine sodium, sodium stearoyl glutamate, coconut fatty acid acylglutamic acid, potassium coconut fatty acid acylglutamate, sodium coconut fatty acid acylglutamate, triethanolamine coconut fatty acid acylglutamate,
lauroyl acylglutamic acid, potassium lauroyl acylglutamate, sodium lauroyl acylglutamate, triethanolamine lauroyl acylglutamate, myristoyl acylglutamic acid, potassium myristoyl acylglutamate, sodium myristoyl acylglutamate, stearoyl acylglutamic acid, potassium stearoyl acylglutamate, disodium stearoyl acylglutamate, sodium hydrogenated tallow fatty acid acylglutamate, sodium coconut fatty acid/hydrogenated tallow fatty acid acylglutamate, methylalanine sodium coconut fatty acid ester, lauroyl methylalanine, lauroyl methylalanine sodium, lauroyl methylalanine triethanolamine, myristoyl methylalanine sodium, lauroyl methyltaurine sodium, methyltaurine potassium coconut fatty acid ester, methyltaurine sodium coconut fatty acid ester, methyltaurine magnesium coconut fatty acid ester,
myristoyl methyltaurine sodium, palmitoyl methyltaurine sodium, stearoyl methyltaurine sodium, oleoyl methyltaurine sodium, sodium alkanesulfonate, sodium tetradecenesulfonate, dioctylsodium sulfosuccinate, lauryl disodium sulfosuccinate, ethyl coconut fatty acid ester sodium sulfonate, sodium laurylsulfate, triethanolamine laurylsulfate, sodium cetyl sulfate, triethanolamine alkylsulfates (11, 13, 15), sodium alkylsulfates (12, 13), triethanolamine alkylsulfates (12, 13), ammonium alkylsulfates (12, 14, 16), diethanolamine alkylsulfates (12, 13), triethanolamine alkylsulfates (12-14), triethanolamine alkylsulfates (12-15), magnesium triethanolamine cocoalkylsulfate,
ammonium laurylsulfate, potassium laurylsulfate, magnesium laurylsulfate, monoethanolamine laurylsulfate, diethanolamine laurylsulfate, sodium myristylsulfate, sodium stearylsulfate, sodium oleylsulfate, triethanolamine oleylsulfate, sodium polyoxyethylene laurylether sulfate, triethanolamine polyoxyethylene laurylether sulfate, sodium polyoxyethylene (1) alkyl (11, 13, 15) ether sulfate, triethanolamine polyoxyethylene (1) alkyl (11, 13, 15) ether sulfate, sodium polyoxyethylene (3) alkyl (11-15) ether sulfate, sodium polyoxyethylene (2) alkyl (12, 13) ether sulfate, sodium polyoxyethylene (3) alkyl (12-14) ether sulfate, sodium polyoxyethylene (3) alkyl (12-15) ether sulfate, sodium polyoxyethylene (2) laurylether sulfate, sodium polyoxyethylene (3) myristylether sulfate, sodium higher fatty acid alkanolamide sulfate,
laurylphosphoric acid, sodium laurylphosphate, potassium cetylphosphate, diethanolamine cetylphosphate, polyoxyethylene oleylether phosphoric acid, polyoxyethylene laurylether phosphoric acid, sodium polyoxyethylene laurylether phosphate, polyoxyethylene cetylether phosphoric acid, sodium polyoxyethylene cetylether phosphate, polyoxyethylene stearylether phosphoric acid, polyoxyethylene oleylether phosphoric acid, sodium polyoxyethylene oleylether phosphate, polyoxyethylene alkylphenyl ether phosphoric acid, sodium polyoxyethylene alkylphenyl ether phosphate, triethanolamine polyoxyethylene alkylphenyl ether phosphate, polyoxyethylene octylether phosphoric acid, polyoxyethylene (10) alkyl (12, 13) ether phosphoric acid, polyoxyethylene alkyl (12-15) ether phosphoric acid, polyoxyethylene alkyl (12-16) ether phosphoric acid, triethanolamine polyoxyethylene laurylether phosphate and diethanolamine polyoxyethylene oleylether phosphate;
cationic surfactants such as dioctylamine, dimethylstearylamine, trilaurylamine, stearic acid diethylaminoethylamide, lauryltrimethylammonium chloride, cetyltrimethylammonium chloride, cetyltrimethylammonium bromide, cetyltrimethylammonium saccharin, stearyltrimethylammonium chloride, alkyl (20-22) trimethylammonium chloride, lauryltrimethylammonium bromide, alkyl (16, 18) trimethylammonium chloride, stearyltrimethylammonium bromide, stearyltrimethylammonium saccharin, alkyl (28) trimethylammonium chloride, di(polyoxyethylene)oleylmethylammonium chloride (2EO), dipolyoxyethylenestearylmethylammonium chloride, polyoxyethylene (1) polyoxypropylene (25) diethylmethylammonium chloride, tri(polyoxyethylene)stearylammonium chloride (5EO), distearyldimethylammonium chloride,
dialkyl (12-15) dimethylammonium chloride, dialkyl (12-18) dimethylammonium chloride, dialkyl (14-18) dimethylammonium chloride, dicocoyldimethylammonium chloride, dicetyldimethylammonium chloride, isostearyllauryldimethylammonium chloride, benzalkonium chloride, myristyldimethylbenzylammonium chloride, lauryldimethyl(ethylbenzyl)ammonium chloride, stearyldimethylbenzylammonium chloride, laurylpyridinium chloride, cetylpyridinium chloride, lauroylcolaminoformylmethylpyridinium chloride, stearoylcolaminoformylmethylpyridinium chloride, alkylisoquinolium bromide, methylbenzethonium chloride and benzethonium chloride;
amphoteric surfactants such as 2₋alkyl-N-carboxymethyl-N-hydroxyethyl imidazolinium betaine, alkyldiaminoethylglycine hydrochloride, lauryldiaminoethylglycine sodium, undecylhydroxyethylimidazolium betaine sodium, undecyl-N-carboxymethylimidazolium betaine, acyl-N-carboxyethyl-N-hydroxyethylethylenediamine disodium coconut fatty acid ester, acyl-N-carboxyethoxyethyl-N-carboxyethylethylenediamine disodium coconut fatty acid ester, acyl-N-carboxymethoxyethyl-N-carboxymethylethylenediamine disodium coconut fatty acid ester,
sodium laurylaminopropionate, sodium laurylaminodipropionate, triethanolamine laurylaminopropionate, acyl-N-carboxyethyl-N-hydroxyethylethylenediamine sodium palm oil fatty acid ester, betaine lauryldimethylaminoacetate, betaine coconut oil alkyldimethylaminoacetate, betaine stearyldimethylaminoacetate, stearyldimethyl betaine sodium, amidopropylbetaine coconut fatty acid ester, amidopropylbetaine palm oil fatty acid ester, lauric acid amide betaine propylacetate, amidopropylbetaine ricinoleate, stearyldihydroxyethylbetaine and laurylhydroxysulfobetaine;
nonionic surfactants such as polyoxyethylene (10) alkyl (12, 13) ether, polyoxyethylene lauryl ether, polyoxyethylene cetyl ether, polyoxyethylene stearyl ether, polyoxyethylene oleyl ether, polyoxyethylene (3, 7, 12) alkyl (12-14) ether, polyoxyethylene tridecyl ether, polyoxyethylene myristyl ether, polyoxyethylene-sec-alkyl (14) ether, polyoxyethylene isocetyl ether, polyoxyethylene cetostearyl ether, polyoxyethylene (2, 10, 20) isostearyl ether, polyoxyethylene oleylcetyl ether, polyoxyethylene (20) aralkyl ether, polyoxyethylene octyldodecyl ether, polyoxyethylene behenyl ether, polyoxyethylene octylphenyl ether, polyoxyethylene nonylphenyl ether, polyoxyethylene dinonylphenyl ether,
polyoxyethylene (1) polyoxypropylene (1, 2, 4, 8) cetyl ether, polyoxyethylene (5) polyoxypropylene (1, 2, 4, 8) cetyl ether, polyoxyethylene (10) polyoxypropylene (1, 2, 4, 8) cetyl ether, polyoxyethylene (20) polyoxypropylene (1, 2, 4, 8) cetyl ether, polyoxyethylene polyoxypropylene lauryl ether, polyoxyethylene (3) polyoxypropylene (34) stearyl ether, polyoxyethylene (4) polyoxypropylene (30) stearyl ether, polyoxyethylene (34) polyoxypropylene (23) stearyl ether, polyoxyethylene polyoxypropylene cetyl ether, polyoxyethylene polyoxypropylene decyltetradecyl ether,
polyethylene glycol monolaurate, ethylene glycol monostearate, polyethylene glycol monostearate, polyethylene glycol monooleate, ethylene glycol fatty acid ester, self-emulsifiable ethylene glycol monostearate, diethylene glycol laurate, polyethylene glycol myristate, polyethylene glycol palmitate, diethylene glycol stearate, self-emulsifiable polyethylene glycol (2) monostearate, polyethylene glycol isostearate, ethylene glycol dioctanoate, diethylene glycol dilaurate, polyethylene glycol dilaurate, polyethylene glycol (150) dipalmitate, ethylene glycol distearate, diethylene glycol distearate, polyethylene glycol distearate, ethylene glycol dioleate, polyethylene glycol dioleate,
polyethylene glycol diricinoleate, polyoxyethylene (20) sorbitan monolaurate, polyoxyethylene (20) sorbitan monopalmitate, polyoxyethylene (6) sorbitan monostearate, polyoxyethylene (20) sorbitan monostearate, polyoxyethylene (20) sorbitan tristearate, polyoxyethylene (6) sorbitan monooleate, polyoxyethylene (20) sorbitan monooleate, polyoxyethylene (20) sorbitan trioleate, sorbitan polyoxyethylene (20) coconut fatty acid ester, polyoxyethylene (10-80) sorbitan monolaurate, polyoxyethylene sorbitan tristearate, polyoxyethylene (20) sorbitan isostearate, polyoxyethylene (150) sorbitan tristearate,
polyoxyethylene castor oil, polyoxyethylene hydrogenated castor oil, polyoxyethylene (10) hydrogenated castor oil, polyoxyethylene (20) hydrogenated castor oil, polyoxyethylene (40) hydrogenated castor oil, polyoxyethylene (50) hydrogenated castor oil, polyoxyethylene (60) hydrogenated castor oil, glyceryl caprylate, glyceryl caprate, glyceryl laurate, glyceryl myristate, glyceryl stearate, glyceryl linoleate, glyceryl oleate, glyceryl isostearate, glyceryl behenate, glyceryl erucate, glyceryl cocofatty acid ester, glyceryl ricinoleate, glyceryl hydroxystearate, wheat germ oil fatty acid monoglyceride, safflower oil fatty acid monoglyceride, hydrogenated soybean fatty acid monoglyceride, saturated fatty acid monoglyceride, cotton seed oil fatty acid monoglyceride, tallow fatty acid monoglyceride, lanolin fatty acid monoglyceride,
glyceryl monoisostearate monomyristate, glyceryl sesquioleate, glyceryl distearate, glyceryl diisostearate, glyceryl diarachidate, sorbitan monolaurate, sorbitan monopalmitate, sorbitan monostearate, sorbitan monoisostearate, sorbitan monooleate, sorbitan sesquistearate, sorbitan sesquioleate, sorbitan tristearate, sorbitan trioleate, sorbitan coconut fatty acid ester, sorbitan isostearate, sorbitan sesquiisostearate, sorbitan distearate, diglyceryl isopalmitate, poly (4-10) glyceryl monolaurate, poly (10) glyceryl monomyristate, poly (2-10) glyceryl monostearate, poly (2-10) glyceryl monoisostearate, poly (2-10) glyceryl monooleate, diglyceryl sesquioleate,
poly (2-10) glyceryl diisostearate, poly (6-10) glyceryl distearate, diglyceryl triisostearate, poly (10) glyceryl tristearate, poly (10) glyceryl trioleate, poly (2) glyceryl tetraisostearate, decaglyceryl pentastearate, poly (6-10) glyceryl pentaoleate, poly (10) glyceryl heptastearate, decaglyceryl decastearate, poly (10) glyceryl decaoleate, condensed poly (6) glyceryl ricinoleate, cane sugar fatty acid ester, cane sugar coconut fatty acid ester, alkyl gluceside, coconut oil alkyldimethylamine oxide, lauryldimethylamine oxide, dihydroxyethyllauryldimethylamine oxide, stearyldimethylamine oxide, oleyldimethylamine oxide and polyoxyethylene coconut oil alkyldimethylamine oxide;
natural surfactants such as saponin, lecithin, soybean phospholipid, hydrogenated soybean phospholipid, soybean lysophospholipid, hydrogenated soybean lysophospholipid, egg yolk lecithin, hydrogenated egg yolk lysophosphatidylcholine, phosphatidylcholine, phosphatidylethanolamine, phosphatidylserine, sphingophospholipid, sphingomyelin, ganglioside, bile acid, cholic acid, deoxycholic acid, sodium cholate, sodium deoxycholate, spiculisporic acid, rhamnolipid, trehalose lipid, sophoroliquid and mannosylerythritol lipid;
ultraviolet light absorbers, including
   paraaminobenzoic acid derivatives such as paraaminobenzoic acid, ethyl paraaminobenzoate, glyceryl paraaminobenzoate, amyl paradimethylaminobenzoate and 2-ethylhexyl paradimethylaminobenzoate,
   cinnamic acid derivatives such as benzyl cinnamate, glyceryl diparamethoxycinnamate mono-2-ethylhexanoate, methyl 2,4-diisopropylcinnamate, ethyl 2,9-diisopropylcinnamate, potassium paramethoxycinnamate, sodium paramethoxycinnamate, isopropyl paramethoxycinnamate, 2-ethylhexyl paramethoxycinnamate, 2-ethoxyethyl paramethoxycinnamate and ethyl paraethoxycinnamate,
   urocanic acid derivatives such as urocanic acid and ethyl urocanate,
benzophenone derivatives such as
   2,4-dihydroxybenzophenone,
   2,2',4,4'-tetrahydroxybenzophenone,
   2-hydroxy-4-methoxy-5-sulfobenzophenonesodium,
   2-hydroxy-4-methoxybenzophenone-5-sulfonic acid,
   2-hydroxy-4-methoxybenzophenone,
   2,2'-dihydroxy-4,4'-dimethoxybenzophenone and
   2,2'-dihydroxy-4,4'-dimethoxy-5-sulfobenzophenonesodium, salicylic acid derivatives such as ethylene glycol salicylate, 2-ethylhexyl salicylate, phenyl salicylate, benzyl salicylate, p-tert-butylphenyl salicylate, homomenthyl salicylate and 3,3,5-trimethylcyclohexyl salicylate,
   2-(2'-hydroxy-5'-methoxyphenyl)benzotriazole and
   4-tert-butyl-4'-methoxybenzoylmethane;
powders and color materials such as kaolin, silicic anhydride, aluminum magnesium silicate, sericite, talc, boron nitride, mica, montmorillonite, hemp cellulose powder, wheat starch, silk powder, cornstarch, natural dyes including nitro dye, azo dye, nitroso dye, triphenylmethane dye, xanthene dye, quinoline dye, anthraquinone dye, indigo dye, pyrene dye, phthalocyanine dye, flavonoid, quinone, porphyrin, water-soluble annatto, squid ink powder, caramel, guaiazulene, gardenia blue, gardenia yellow, cochineal, shikonin, copper chlorophyllin sodium, paprika dye, safflower red, safflower yellow, laccaic acid and riboflavin butyrate, carbon black, yellow iron oxide, black iron oxide, red iron oxide, iron blue, ultramarine blue, zinc oxide, chromium oxide, titanium oxide, black titanium oxide, zirconium oxide, chromium hydroxide, alumina, magnesium oxide, barium sulfate, aluminum hydroxide, calcium carbonate, lithium cobalt titanate, manganese violet and pearl pigment;
plant extracts such as angelica keiskei extract, gambir extract, avocado extract, hydrangea serrata leaf extract, gynostemma pentaphyllum extract, althea extract, arnica extract, oil-soluble arnica extract, almond extract, aloe extract, styrax benzoin resin extract, ginkgo extract, urtica extract, orris root extract, fennel extract, turmeric extract, rose fruit extract, echinacea leaf extract, scutellaria baicalensis root extract, phellodendron bark extract, coptis rhizome extract, hordeum vulgare seed extract, gumbo extract, hypericum erectum extract, oil-soluble hypericum erectum extract, lamium album flower extract, oil-soluble lamium album flower extract, ononis extract, nasturtium officinale extract, orange extract, orange flower water, seaweed extract, kaki tannin, puerariae radix extract, valerian extract, cattail extract, chamomilla extract, oil-soluble chamomilla extract, chamomilla water, oat extract, carrot extract, oil-soluble carrot extract, carrot oil, artemisia capillaris extract,
licorice extract, licorice extract powder, licorice flavonoid, cantharis tincture, raspberry extract, kiwi extract, cinchona bark extract, cucumber extract, apricot kernel extract, quince seed extract, gardenia extract, sasa veitchii extract, sophora angustifolia extract, walnut shell extract, grapefruit extract, clematis extract, brown sugar extract, chlorella extract, mulberry extract, cinnamon bark extract, gentian extract, geranium herb extract, tea extract, spatterdock extract, arctium lappa root extract, oil-soluble arctium lappa root extract, wheat germ extract, hydrolyzed wheat powder, rice bran extract, rice bran fermentation extract, comfrey extract, asiasarum root extract, saffron extract, saponaria officinalis extract, oil-soluble salvia extract, crataegus cuneata fruit extract, xanthoxylum extract, shiitake mushroom extract, shiitake mushroom extract powder, rehmannia glutinosa extract, sycon extract, oil-soluble sycon extract, Japanese basil extract, linden extract, oil-soluble linden extract,
filipendula multijuga extract, crude drug extract, coix lacryma-jobi seed extract, ginger extract, oil-soluble ginger extract, ginger tincture, acorus calamus root extract, betula alba extract, oil-soluble betula alba extract, betula alba sap, lonicera extract, equisetum arvense extract, oil-soluble equisetum arvense extract, scordinin, stevia extract, ivy extract, crataegus oxyacantha extract, sambucus nigra flower extract, juniperus communis extract, achillea millefolium extract, oil-soluble achillea millefolium extract, mentha piperita extract, sage extract, oil-soluble sage extract, sage water, malva sylvestris extract, celery extract, cnidium officinale extract, cnidium officinale water, swertia japonica extract, soybean extract, jujube extract, thyme extract, camellia sinensis leaf extract, camellia sinensis dry distillate, camellia sinensis seed extract, clove flower extract, citrus unshiu peel extract, camellia japonica seed extract, centella asiatica extract, oil-soluble juglans regia extract, duke extract, terminalia extract, red pepper tincture,
angelica acutiloba extract, oil-soluble angelica acutiloba extract, angelica acutiloba water, calendula officinalis flower extract, oil-soluble calendula officinalis flower extract, soymilk powder, prunus persica extract, citrus aurantium amara extract, houttuynia cordata extract, tomato extract, potentilla erecta root extract, natto extract, ginseng extract, oil-soluble ginseng extract, garlic extract, rosa canina fruit extract, oil-soluble rosa canina fruit extract, malt extract, malt root extract, ophiopogon tuber extract, parsley extract, hordeum vulgare leaf juice concentrate, distilled peppermint water, hamamelis water, hamamelis extract, rosa centifolia flower extract, parietaria extract, isodonis japonicus extract, eriobotrya japonica leaf extract, oil-soluble eriobotrya japonica leaf extract, coltsfoot flower extract, poria cocos extract, ruscus aculeatus root extract, ruscus aculeatus root extract powder, grape extract, grape leaf extract, grape water, hayflower extract, luffa cylindrica fruit extract, luffa cylindrica fruit water, safflower extract, oil-soluble tilia miqueliana extract, tilia miqueliana water, paeonia suffruticosa root extract, hops extract, oil-soluble hops extract,
pinus sylvestris cone extract, silybum marianum fruit extract, horse chestnut extract, oil-soluble horse chestnut extract, sapindus mukurossi peel extract, melissa officinalis leaf extract, melilotus officinalis extract, peach leaf extract, oil-soluble peach leaf extract, bean-sprouts extract, centaurea cyanus flower extract, centaurea cyanus flower water, eucalyptus extract, saxifraga sarmentosa extract, lilium candidum bulb extract, coix lacryma jobi seed extract, oil-soluble coix lacryma jobi seed extract, artemisia princeps extract, artemisia princeps water, lavender extract, lavender water, apple extract, ganoderma lucidum extract, lettuce extract, lemon extract, astragalus sinicus extract, rose water, rosemary extract, oil-soluble rosemary extract, anthemis nobilis flower extract and sanguisorba officinalis root extract;
amino acids and peptides such as glycine, alanine, valise, leucine, isoleucine, serine, threonine, phenylalanine, tyrosine, tryptophan, cystine, cysteine, methionine, proline, hydroxyproline, aspartic acid, asparagine, glutamic acid, glutamine, arginine, histidine, lysine, γ-aminobutyric acid, DL-pyrrolidonecarboxylic acid, ε-aminocaproic acid, hydrolyzed elastin, water-soluble elastin, hydrolyzed collagen, water-soluble collagen, casein, glutathione, wheat peptide and soybean peptide;
vitamins and vitamin affecters, including
   vitamin A such as retinol, retinal, retinoic acid, retinol acetate and retinol palmitate,
   carotenoids such as α-carotene, β-carotene, γ-carotene, δ-carotene, lycopene, zeaxanthin, cryptoxanthin, echinenone and astaxanthin,
   vitamin B1 such as thiamines,
   vitamin B2 such as riboflavin,
   vitamin B6 such as pyridoxine, pyridoxal and pyridoxamine, vitamin B12 such as cyanocobalamin,
   folic acids, nicotinic acid, nicotinic acid amide, pantothenic acids, biotins,
   vitamin C such as L-ascorbic acid, sodium L-ascorbate, L-ascorbyl stearate,
L-ascorbyl palmitate, L-ascorbyl dipalmitate, L-ascorbyl tetraisopalmitate, disodium L-ascorbate sulfate, magnesium L-ascorbate, L-ascorbyl sodium phosphate and L-ascorbic acid-2-glucoside,
   vitamin D such as ergocalciferol and cholecalciferol, vitamin E such as d-α-tocopherol, DL-α-tocopherol, dl-α-tocopherol acetate, dl-α-tocopherol succinate, β-tocopherol, γ-tocopherol and d-δ-tocopherol, ubiquinones, vitamin K, carnitine, ferulic acid, γ-oryzanol, α-lipoic acid and orotic acid;
antiseptics such as benzoic acid, sodium benzoate, undecylenic acid, salicylic acid, sorbic acid, potassium sorbate, dehydroacetic acid, sodium dehydroacetate, isobutyl parahydroxybenzoate, isopropyl parahydroxybenzoate, ethyl parahydroxybenzoate, butyl parahydroxybenzoate, propyl parahydroxybenzoate, benzyl parahydroxybenzoate, methyl parahydroxybenzoate, methyl sodium parahydroxybenzoate, phenoxyethanol, photosensitive agent No. 101, photosensitive agent No. 201 and photosensitive agent No. 401;
antioxidants such as butylhydroxyanisole, butylhydroxytoluene, propyl gallate, erythorbic acid, sodium erythorbate, parahydroxyanisole and octyl gallate;
sequestering agents such as trisodium ethylenediaminehydroxyethyltriacetate, edeticacid, disodium edetate, trisodium edetate, tetrasodium edetate, sodium citrate, gluconic acid, phytic acid, sodium polyphosphate and sodium metaphosphate;
moisturizers such as hyaluronic acid, sodium hyaluronate, sodium chondroitinsulfate, sodium lactate, sodium pyrrolidonecarboxylate, betaine, lactic acid bacteria culture solution, yeast extract and ceramide;
anti-inflammatory agents such as glycyrrhizinic acid, trisodium glycyrrhizinate, dipotassium glycyrrhizinate, monoammonium glycyrrhizinate, β-glycyrrhetinic acid, glyceryl glycyrrhetinate, stearyl glycyrrhetinate, lysozyme chloride, hydrocortisone and allantoin;
pH adjusters such as sodium hydroxide, potassium hydroxide and triethanolamine;
salts such as sodium chloride, potassium chloride, magnesium chloride and sodium sulfate;
α-hydroxy acids such as citric acid, glycolic acid, tartaric acid and lactic acid;
whitening agents such as arbutin, α-arbutin and placental extract;
essential oils such as angelica oil, ylang ylang oil, elemi oil, orange oil, German chamomile oil, anthemis nobilis oil, cardamom oil, calamus oil, galbanum oil, camphor oil, carrot seed oil, clary sage oil, grapefruit oil, clove oil, cinnamon bark oil, coriander oil, cypress oil, sandalwood oil, cedarwood oil, citronella oil, cinnamon leaf oil, jasmine absolute, juniper berry oil, ginger extract, spearmint oil, sage oil, cedar oil, geranium oil, thyme oil, tea tree oil, nutmeg oil, niaouli oil, neroli oil, pine oil, basil oil, peppermint oil, patchouli oil, palmarosa oil, fennel oil, petitgrain oil, black pepper oil, frankincense oil, vetivert oil; peppermint oil, bergamot oil, benzoin oil, aniba rosaeodora oil, marjoram oil, mandarin oil, myrrh oil, melissa oil, eucalyptus oil, Chinese lemon oil, lime oil, ravensara oil, lavandin oil, lavender oil, lindane oil, lemon oil, lemongrass oil, rose oil, rosewood oil, rosemary oil and lovage oil;
terpenes such as limonene, pinene, terpinene, terpinolene, myrcene and longifolene;
perfumes and water.

### <Emulsified cosmetic>

The emulsified skin external preparation contains the tocopherol glycinate derivative, at least one fatty acid ester selected from the glycerin mono fatty acid esters, polyglycerin fatty acid esters and dextrin fatty acid esters, and the additional components described above. Emulsified cosmetics are the most frequent form of emulsified skin external preparations.

The emulsified cosmetics are not particularly limited as long as they are used directly on skin, and examples thereof include milky lotions, sera, creams and gels. They may be used regardless of user's gender and age.

The emulsified cosmetic according to the present invention may contain existing cosmetic ingredients while still achieving the effects of the invention.

The existing cosmetic ingredients include those listed in the following documents: The Japanese Standards of Cosmetic Ingredients 2nd edition (edited by Society of Japanese Pharmacopoeia and published by Yakuji Nippo, Ltd. (1984)), The Japanese Cosmetic Ingredients Codex (edited by Ministry of Health and Welfare, Pharmaceutical Examination Division and published by Yakuji Nippo, Ltd. (1993)), Supplement to The Japanese Cosmetic Ingredients Codex (edited by Ministry of Health and Welfare, Pharmaceutical Examination Division and published by Yakuji Nippo, Ltd. (1993)), The Comprehensive Licensing Standards of Cosmetics by Category (edited by Ministry of Health and Welfare, Pharmaceutical Examination Division and published by Yakuji Nippo, Ltd. (1993)), The Japanese Cosmetic Ingredients Codex by Category (edited by Ministry of Health and Welfare, Pharmaceutical Examination Division and published by Yakuji Nippo, Ltd. (1997)), and Dictionary of Cosmetic Ingredients (Nikko Chemicals., Co. Ltd. (1991)).

The emulsified skin external preparation and emulsified cosmetic of the present invention may be produced by common methods depending on the formulations, for example by dissolving, mixing or dispersing the aforesaid ingredients in predetermined amounts.

### <Method for stabilizing emulsified skin external preparation>

The emulsified skin external preparation of the invention contains the tocopherol glycinate derivative in combination with at least one fatty acid ester selected from the glycerin mono fatty acid esters, polyglycerin fatty acid esters and dextrin fatty acid esters, whereby the viscosity reduction and separation during storage are prevented.

Specifically, the combined use of the tocopherol glycinate derivative and at least one fatty acid ester selected from the glycerin mono fatty acid esters, polyglycerin fatty acid esters and dextrin fatty acid esters stabilizes the skin external preparation containing the tocopherol glycinate derivative. This method for stabilizing the skin external preparation containing the tocopherol glycinate derivative is an aspect of the present invention.

### EXAMPLES

Hereinbelow, the present invention will be described in greater detail by examples. However, it should be construed that the invention is not limited thereto.

The glycerin mono fatty acid esters used in Examples were glyceryl stearate, glyceryl isostearate, glyceryl laurate, glyceryl myristate, glyceryl oleate and glyceryl ricinoleate. The polyglycerin fatty acid esters used in Examples were poly (10) glyceryl monostearate, poly (10) glyceryl monoisostearate and poly (10) glyceryl myristate. The dextrin fatty acid esters used in Examples were dextrin stearate, dextrin palmitate and dextrin myristate. The glycerin used in Examples had a purity of at least 98% by mass. The storage stability was tested as follows.

### <Storage stability test>

The sample was placed in a glass bottle and was allowed to stand at 40°C for a month. The state and appearance were visually observed. In addition, viscosity was measured before and after the test. Samples having no defect were evaluated as "AA" and samples having defects (such as separation and fluidization due to viscosity reduction) were evaluated as "BB". The viscosity was measured with the E type viscometer (VISCONIC EHD manufactured by Tokyo Keiki Co., Ltd., rotor No. 4) using 1 g of the sample under the condition of 25°C and 0.5 rpm.

### [Examples 1-7 and Comparative Examples 1-2]

The materials of component I were mixed together in the amounts shown in Table 1 at 80°C and were dissolved.
Separately, the materials of component II were mixed together in the amounts shown in Table 1 at 80°C and were dissolved. The component II was added to the component I. The mixture was cooled with stirring and was emulsified. The emulsion was cooled to 30°C to give a cream. The cream was tested for storage stability. The results are shown in Table 1, in which the values are % by mass except for the viscosity value.

**Table 1**

| | | Ex. | | | | | | | Comp. Ex. | |
|---|---|---|---|---|---|---|---|---|---|---|
| | | 1 | 2 | 3 | 4 | 5 | 6 | 7 | 1 | 2 |
| I | Glyceryl stearate | 0.1 | 0.2 | 1.0 | 2.0 | 5.0 | 10.0 | 20.0 | | |
| | Stearic acid | | | | | | | | | 3.0 |
| | Hydrogenated rapeseed oil alcohol | 4.2 | 4.2 | 4.2 | 4.2 | 4.2 | 4.2 | 4.2 | 4.2 | 4.2 |
| | Isononyl isononanoate | 6.0 | 6.0 | 6.0 | 6.0 | 6.0 | 6.0 | 6.0 | 6.0 | 6.0 |
| | Squalane | 9.6 | 9.6 | 9.6 | 9.6 | 9.6 | 9.6 | 9.6 | 9.6 | 9.6 |
| | Octyldodecyl myristate | 4.8 | 4.8 | 4.8 | 4.8 | 4.8 | 4.8 | 4.8 | 4.8 | 4.8 |
| | POE (20) sorbitan stearate | 1.0 | 1.0 | 1.0 | 1.0 | 1.0 | 1.0 | 1.0 | 1.0 | 1.0 |
| | Tocopherol | 0.2 | 0.2 | 0.2 | 0.2 | 0.2 | 0.2 | 0.2 | 0.2 | 0.2 |
| | Propylparaben | 0.1 | 0.1 | 0.1 | 0.1 | 0.1 | 0.1 | 0.1 | 0.1 | 0.1 |
| | Xanthan gum | 0.1 | 0.1 | 0.1 | 0.1 | 0.1 | 0.1 | 0.1 | 0.1 | 0.1 |
| | dl-α-Tocopherol dimethylglycinate | 1.0 | 1.0 | 1.0 | 1.0 | 1.0 | 1.0 | 1.0 | 1.0 | 1.0 |
| II | 1,3-BG | 4.8 | 4.8 | 4.8 | 4.8 | 4.8 | 4.8 | 4.8 | 4.8 | 4.8 |
| | Glycerin | 4.8 | 4.8 | 4.8 | 4.8 | 4.8 | 4.8 | 4.8 | 4.8 | 4.8 |
| | Trisodium citrate | 0.4 | 0.4 | 0.4 | 0.4 | 0.4 | 0.4 | 0.4 | 0.4 | 0.4 |
| | Potassium hydroxide | | | | | | | | | 0.2 |
| | Purified water | 62.9 | 62.8 | 62.0 | 61.0 | 58.0 | 53.0 | 43.0 | 63.0 | 59.8 |
| Storage stability test | | AA | AA | AA | AA | AA | AA | AA | BB | BB |
| Viscosity before test [mPa·s] | | 15000 | 18000 | 23000 | 26000 | 30000 | 37000 | 40000 | 12000 | 20000 |
| Viscosity after test [mPa·s] | | 14000 | 17000 | 23000 | 26000 | 29000 | 36000 | 39000 | 7000 | 11000 |

As shown in Table 1, Examples 1 to 7 used glyceryl stearate, whilst Comparative Example 1 did not use glyceryl stearate and Comparative Example 2 replaced glyceryl stearate by stearic acid and potassium hydroxide. The creams of Examples 1 to 7 were shown to have superior storage stability to the creams of Comparative Examples 1 and 2.

### [Examples 8-15]

Creams were prepared as described above using the materials in the amounts shown in Table 2. The creams were tested for storage stability. The results are shown in Table 2, in which the values are % by mass except for the viscosity value.

**Table 2**

| | | Ex. | | | | | | | |
|---|---|---|---|---|---|---|---|---|---|
| | | 8 | 9 | 10 | 11 | 12 | 13 | 14 | 15 |
| I | Glyceryl stearate | 3.0 | 3.0 | 3.0 | 3.0 | 3.0 | 3.0 | 3.0 | 3.0 |
| | Hydrogenated rapeseed oil alcohol | 6.3 | 6.3 | 6.3 | 6.3 | 6.3 | 6.3 | 6.3 | 6.3 |
| | Isononyl isononanoate | 3.0 | 3.0 | 3.0 | 3.0 | 3.0 | 3.0 | 3.0 | 3.0 |
| | Squalane | 4.8 | 4.8 | 4.8 | 4.8 | 4.8 | 4.8 | 4.8 | 4.8 |
| | Octyldodecyl myristate | 9.6 | 9.6 | 9.6 | 9.6 | 9.6 | 9.6 | 9.6 | 9.6 |
| | POE (20) sorbitan stearate | 4.0 | 4.0 | 4.0 | 4.0 | 4.0 | 4.0 | 4.0 | 4.0 |
| | Tocopherol acetate | 0.1 | 0.1 | 0.1 | 0.1 | 0.1 | 0.1 | 0.1 | 0.1 |
| | Methylparaben | 0.1 | 0.1 | 0.1 | 0.1 | 0.1 | 0.1 | 0.1 | 0.1 |
| | Hydroxyethyl cellulose | 0.1 | 0.1 | 0.1 | 0.1 | 0.1 | 0.1 | 0.1 | 0.1 |
| | dl-α-Tocopherol dimethylglycinate | 0.05 | 0.1 | 0.5 | 1.0 | 2.0 | 5.0 | 10.0 | 20.0 |
| II | 1,3-BG | 2.4 | 2.4 | 2.4 | 2.4 | 2.4 | 2.4 | 2.4 | 2.4 |
| | Glycerin | 6.0 | 6.0 | 6.0 | 6.0 | 6.0 | 6.0 | 6.0 | 6.0 |
| | Trisodium citrate | 0.1 | 0.1 | 0.2 | 0.4 | 0.6 | 0.6 | 0.6 | 0.6 |
| | Purified water | 60.5 | 60.4 | 59.9 | 59.2 | 58.0 | 55.0 | 50.0 | 40.0 |
| Storage stability test | | AA | AA | AA | AA | AA | AA | AA | AA |
| Viscosity before test [mPa·s] | | 27000 | 27000 | 27000 | 27000 | 27000 | 26000 | 26000 | 25000 |
| Viscosity after test [mPa·s] | | 27000 | 27000 | 27000 | 27000 | 27000 | 26000 | 25000 | 24000 |

As shown in Table 2, the creams of Examples 8 to 15 were shown to have excellent storage stability.

### [Examples 16-24]

Creams were prepared as described above using the materials in the amounts shown in Table 3. The creams were tested for storage stability. The results are shown in Table 3, in which the values are % by mass except for the viscosity value.

**Table 3**

| | | Ex. | | | | | | | | |
|---|---|---|---|---|---|---|---|---|---|---|
| | | 16 | 17 | 18 | 19 | 20 | 21 | 22 | 23 | 24 |
| I | Glyceryl laurate | | | 1.0 | 1.0 | | | | | |
| | Glyceryl myristate | 1.0 | | | | 1.0 | 1.0 | 1.0 | | |
| | Glyceryl stearate | | 1.0 | 1.0 | | 1.0 | | | 1.0 | 1.0 |
| | Glyceryl isostearate | | | | 1.0 | | 1.0 | | | |
| | Glyceryl oleate | | | | | | | 1.0 | 1.0 | |
| | Glyceryl ricinoleate | | | | | | | | | 1.0 |
| | Hydrogenated rapeseed oil alcohol | 4.2 | 4.2 | 4.2 | 4.2 | 4.2 | 4.2 | 4.2 | 4.2 | 4.2 |
| | Isononyl isononanoate | 6.0 | 6.0 | 6.0 | 6.0 | 6.0 | 6.0 | 6.0 | 6.0 | 6.0 |
| | Squalane | 9.6 | 9.6 | 9.6 | 9.6 | 9.6 | 9.6 | 9.6 | 9.6 | 9.6 |
| | Octyldodecyl myristate | 4.8 | 4.8 | 4.8 | 4.8 | 4.8 | 4.8 | 4.8 | 4.8 | 4.8 |
| | Poly (10) glyceryl monostearate | 2.0 | 2.0 | 2.0 | 2.0 | 2.0 | 2.0 | 2.0 | 2.0 | 2.0 |
| | Propylparaben | 0.1 | 0.1 | 0.1 | 0.1 | 0.1 | 0.1 | 0.1 | 0.1 | 0.1 |
| | Xanthan gum | 0.1 | 0.1 | 0.1 | 0.1 | 0.1 | 0.1 | 0.1 | 0.1 | 0.1 |
| | dl-α-Tocopherol dimethylglycinate | 1.0 | 1.0 | 1.0 | 1.0 | 1.0 | 1.0 | 1.0 | 1.0 | 1.0 |
| II | 1,3-BG | 4.8 | 4.8 | 4.8 | 4.8 | 4.8 | 4.8 | 4.8 | 4.8 | 4.8 |
| | Glycerin | 4.8 | 4.8 | 4.8 | 4.8 | 4.8 | 4.8 | 4.8 | 4.8 | 4.8 |
| | Trisodium citrate | 0.4 | 0.4 | 0.4 | 0.4 | 0.4 | 0.4 | 0.4 | 0.4 | 0.4 |
| | Purified water | 61.2 | 61.2 | 60.2 | 60.2 | 60.2 | 60.2 | 60.2 | 60.2 | 60.2 |
| Storage stability test | | AA | AA | AA | AA | AA | AA | AA | AA | AA |
| Viscosity before test [mPa·s] | | 22000 | 22000 | 24000 | 24000 | 24000 | 24000 | 24000 | 24000 | 24000 |
| Viscosity after test [mPa·s] | | 22000 | 22000 | 24000 | 24000 | 24000 | 24000 | 24000 | 24000 | 24000 |

As shown in Table 3, the creams of Examples 16 to 24 were shown to have excellent storage stability.

### [Examples 25-31 and Comparative Examples 3-4]

Creams were prepared as described above using the materials in the amounts shown in Table 4. The creams were tested for storage stability. The results are shown in Table 4, in which the values are % by mass except for the viscosity value.

**Table 4**

| | | Ex. | | | | | | | Comp. Ex. | |
|---|---|---|---|---|---|---|---|---|---|---|
| | | 25 | 26 | 27 | 28 | 29 | 30 | 31 | 3 | 4 |
| I | Dextrin palmitate | 0.1 | 0.5 | 1.0 | 2.0 | 5.0 | 10.0 | 20.0 | | |
| | Palmitic acid | | | | | | | | | 2.4 |
| | Behenyl alcohol | 3.2 | 3.2 | 3.2 | 3.2 | 3.2 | 3.2 | 3.2 | 3.2 | 3.2 |
| | Seaweed extract | 0.1 | 0.1 | 0.1 | 0.1 | 0.1 | 0.1 | 0.1 | 0.1 | 0.1 |
| | Xanthan gum | 0.2 | 0.2 | 0.2 | 0.2 | 0.2 | 0.2 | 0.2 | 0.2 | 0.2 |
| | Methylparaben | 0.1 | 0.1 | 0.1 | 0.1 | 0.1 | 0.1 | 0.1 | 0.1 | 0.1 |
| | Propylparaben | 0.1 | 0.1 | 0.1 | 0.1 | 0.1 | 0.1 | 0.1 | 0.1 | 0.1 |
| | Stearyl glycyrrhizinate | 0.1 | 0.1 | 0.1 | 0.1 | 0.1 | 0.1 | 0.1 | 0.1 | 0.1 |
| | Dimethicone | 2.4 | 2.4 | 2.4 | 2.4 | 2.4 | 2.4 | 2.4 | 2.4 | 2.4 |
| | Squalane | 4.8 | 4.8 | 4.8 | 4.8 | 4.8 | 4.8 | 4.8 | 4.8 | 4.8 |
| | Isononyl isononanoate | 2.4 | 2.4 | 2.4 | 2.4 | 2.4 | 2.4 | 2.4 | 2.4 | 2.4 |
| | Tocopherol | 0.2 | 0.2 | 0.2 | 0.2 | 0.2 | 0.2 | 0.2 | 0.2 | 0.2 |
| | Trioctanoin | 2.4 | 2.4 | 2.4 | 2.4 | 2.4 | 2.4 | 2.4 | 2.4 | 2.4 |
| | 2-Octyldodecanol | 2.4 | 2.4 | 2.4 | 2.4 | 2.4 | 2.4 | 2.4 | 2.4 | 2.4 |
| | POE (20) sorbitan stearate | 4.0 | 4.0 | 4.0 | 4.0 | 4.0 | 4.0 | 4.0 | 4.0 | 4.0 |
| | 1,3-Butylene glycol | 2.4 | 2.4 | 2.4 | 2.4 | 2.4 | 2.4 | 2.4 | 2.4 | 2.4 |
| | Glycerin | 4.8 | 4.8 | 4.8 | 4.8 | 4.8 | 4.8 | 4.8 | 4.8 | 4.8 |
| | Sodium hyaluronate (1% aqueous solution) | 4.8 | 4.8 | 4.8 | 4.8 | 4.8 | 4.8 | 4.8 | 4.8 | 4.8 |
| II | dl-α-Tocopherol dimethylglycinate | 1.0 | 1.0 | 1.0 | 1.0 | 1.0 | 1.0 | 1.0 | 1.0 | 1.0 |
| | Trisodium citrate | 0.4 | 0.4 | 0.4 | 0.4 | 0.4 | 0.4 | 0.4 | 0.4 | 0.4 |
| | Sodium chloride | 0.8 | 0.8 | 0.8 | 0.8 | 0.8 | 0.8 | 0.8 | 0.8 | 0.8 |
| | Potassium hydroxide | | | | | | | | | 0.1 |
| | Purified water | 63.3 | 62.9 | 62.4 | 61.4 | 58.4 | 53.4 | 43.4 | 63.4 | 60.9 |
| Storage stability test | | AA | AA | AA | AA | AA | AA | AA | BB | BB |
| Viscosity before test [mPa·s] | | 21000 | 26000 | 36000 | 43000 | 47000 | 49000 | 50000 | 20000 | 18000 |
| Viscosity after test [mPa·s] | | 18000 | 24000 | 36000 | 43000 | 47000 | 49000 | 50000 | 12000 | 9000 |

As shown in Table 4, Examples 25 to 31 used dextrin palmitate, whilst Comparative Example 3 did not use dextrin palmitate and Comparative Example 4 replaced dextrin palmitate by palmitic acid and potassium hydroxide. The creams of Examples 25 to 31 were shown to have superior storage stability to the creams of Comparative Examples 3 and 4.

### [Examples 32-39]

Creams were prepared as described above using the materials in the amounts shown in Table 5. The creams were tested for storage stability. The results are shown in Table 5, in which the values are % by mass except for the viscosity value.

**Table 5**

| | | Ex. | | | | | | | |
|---|---|---|---|---|---|---|---|---|---|
| | | 32 | 33 | 34 | 35 | 36 | 37 | 38 | 39 |
| I | Dextrin palmitate | 2.4 | 2.4 | 2.4 | 2.4 | 2.4 | 2.4 | 2.4 | 2.4 |
| | Behenyl alcohol | 3.2 | 3.2 | 3.2 | 3.2 | 3.2 | 3.2 | 3.2 | 3.2 |
| | Seaweed extract | 0.1 | 0.1 | 0.1 | 0.1 | 0.1 | 0.1 | 0.1 | 0.1 |
| | Xanthan gum | 0.2 | 0.2 | 0.2 | 0.2 | 0.2 | 0.2 | 0.2 | 0.2 |
| | Methylparaben | 0.1 | 0.1 | 0.1 | 0.1 | 0.1 | 0.1 | 0.1 | 0.1 |
| | Propylparaben | 0.1 | 0.1 | 0.1 | 0.1 | 0.1 | 0.1 | 0.1 | 0.1 |
| | Stearyl glycyrrhizinate | 0.1 | 0.1 | 0.1 | 0.1 | 0.1 | 0.1 | 0.1 | 0.1 |
| | Dimethicone | 2.4 | 2.4 | 2.4 | 2.4 | 2.4 | 2.4 | 2.4 | 2.4 |
| | Squalane | 4.8 | 4.8 | 4.8 | 4.8 | 4.8 | 4.8 | 4.8 | 4.8 |
| | Isononyl isononanoate | 2.4 | 2.4 | 2.4 | 2.4 | 2.4 | 2.4 | 2.4 | 2.4 |
| | Tocopherol | 0.2 | 0.2 | 0.2 | 0.2 | 0.2 | 0.2 | 0.2 | 0.2 |
| | Trioctanoin | 2.4 | 2.4 | 2.4 | 2.4 | 2.4 | 2.4 | 2.4 | 2.4 |
| | 2-Octyldodecanol | 2.4 | 2.4 | 2.4 | 2.4 | 2.4 | 2.4 | 2.4 | 2.4 |
| | Poly (10) glyceryl monostearate | 12.0 | 12.0 | 12.0 | 12.0 | 12.0 | 12.0 | 12.0 | 12.0 |
| | 1,3-Butylene glycol | 2.4 | 2.4 | 2.4 | 2.4 | 2.4 | 2.4 | 2.4 | 2.4 |
| | Glycerin | 4.8 | 4.8 | 4.8 | 4.8 | 4.8 | 4.8 | 4.8 | 4.8 |
| | Sodium hyaluronate (1% aqueous solution) | 4.8 | 4.8 | 4.8 | 4.8 | 4.8 | 4.8 | 4.8 | 4.8 |
| II | dl-α-Tocopherol dimethylglycinate | 0.05 | 0.1 | 0.5 | 1.0 | 2.0 | 5.0 | 10.0 | 20.0 |
| | Trisodium citrate | 0.4 | 0.4 | 0.4 | 0.4 | 0.4 | 0.4 | 0.4 | 0.4 |
| | Sodium chloride | 0.8 | .0.8 | 0.8 | 0.8 | 0.8 | 0.8 | 0.8 | 0.8 |
| | Purified water . | 54.0 | 53.9 | 53.5 | 53.0 | 52.0 | 49.0 | 44.0 | 34.0 |
| Storage stability test | | AA | AA | AA | AA | AA | AA | AA | AA |
| Viscosity before test [mPa·s] | | 45000 | 45000 | 45000 | 45000 | 45000 | 43000 | 40000 | 39000 |
| Viscosity after test [mPa·s] | | 45000 | 45000 | 45000 | 45000 | 45000 | 42000 | 38000 | 36000 |

As shown in Table 5, the creams of Examples 32 to 39 were shown to have excellent storage stability.

### [Examples 40-42]

Creams were prepared as described above using the materials in the amounts shown in Table 6. The creams were tested for storage stability. The results are shown in Table 6, in which the values are % by mass except for the viscosity value.

**Table 6**

| | | Ex. | | |
|---|---|---|---|---|
| | | 40 | 41 | 42 |
| I | Dextrin myristate | 2.4 | 2.4 | |
| | Dextrin palmitate | 2.4 | | 2.4 |
| | Dextrin stearate | | 2.4 | 2.4 |
| | Glyceryl stearate | 2.0 | 2.0 | 2.0 |
| | Stearic acid | 1.2 | 1.2 | 1.2 |
| | Cetostearyl alcohol | 1.2 | 1.2 | 1.2 |
| | Tea tree oil | 0.1 | 0.1 | 0.1 |
| | Synthetic sodium magnesium silicate | 0.4 | 0.4 | 0.4 |
| | Dipotassium glycyrrhizinate | 0.1 | 0.1 | 0.1 |
| | Dicapryl carbonate | 2.4 | 2.4 | 2.4 |
| | Olive oil | 4.8 | 4.8 | 4.8 |
| | Octyldodecyl neopentanoate | 4.8 | 4.8 | 4.8 |
| | Tocopherol | 0.1 | 0.1 | 0.1 |
| | Jojoba oil | 4.8 | 4.8 | 4.8 |
| | 2-Octyldodecanol | 2.4 | 2.4 | 2.4 |
| | POE (20) sorbitan stearate | 4.8 | 4.8 | 4.8 |
| | 1,2-Hexanediol | 2.4 | 2.4 | 2.4 |
| | Glycerin | 4.8 | 4.8 | 4.8 |
| | Polyquaternium 51 | 0.3 | 0.3 | 0.3 |
| | Polyvinylpyrrolidone | 0.1 | 0.1 | 0.1 |
| | Acrylate/alkyl (C10-30) acrylate cross polymer | 0.2 | 0.2 | 0.2 |
| II | dl-α-tocopherol dimethylglycinate | 1.0 | 1.0 | 1.0 |
| | dl-α-tocopherol sarcosinate | 1.0 | 1.0 | 1.0 |
| | Sodium lactate | 0.4 | 0.4 | 0.4 |
| | Sodium tartrate | 0.4 | 0.4 | 0.4 |
| | Sodium pyrrolidonecarboxylate | 0.2 | 0.2 | 0.2 |
| | Arginine | 0.1 | 0.1 | 0.1 |
| | Serine | 0.1 | 0.1 | 0.1 |
| | Tetrasodium hydroxyethane diphosphonate | 0.4 | 0.4 | 0.4 |
| | Purified water | 59.5 | 59.5 | 59.5 |
| Storage stability test | | AA | AA | AA |
| Viscosity before test [mPa·s] | | 50000 | 50000 | 50000 |
| Viscosity after test [mPa·s] | | 50000 | 50000 | 50000 |

As shown in Table 6, the creams of Examples 40 to 42 were shown to have excellent storage stability.

### [Examples 43-49 and Comparative Examples 5-6]

Creams were prepared as described above using the materials in the amounts shown in Table 7. The creams were tested for storage stability. The results are shown in Table 7, in which the values are % by mass except for the viscosity value.

**Table 7**

| | | Ex. | | | | | | | Comp. Ex. | |
|---|---|---|---|---|---|---|---|---|---|---|
| | | 43 | 44 | 45 | 46 | 47 | 48 | 49 | 5 | 6 |
| I | Dextrin palmitate | 2.4 | 2.4 | 2.4 | 2.4 | 2.4 | 2.4 | 2.4 | | |
| | Palmitic acid | | | | | | | | | 2.4 |
| | Behenyl alcohol | 3.2 | 3.2 | 3.2 | 3.2 | 3.2 | 3.2 | 3.2 | 3.2 | 3.2 |
| | Seaweed extract | 0.1 | 0.1 | 0.1 | 0.1 | 0.1 | 0.1 | 0.1 | 0.1 | 0.1 |
| | Xanthan gum | 0.2 | 0.2 | 0.2 | 0.2 | 0.2 | 0.2 | 0.2 | 0.2 | 0.2 |
| | Methylparaben | 0.1 | 0.1 | 0.1 | 0.1 | 0.1 | 0.1 | 0.1 | 0.1 | 0.1 |
| | Propylparaben | 0.1 | 0.1 | 0.1 | 0.1 | 0.1 | 0.1 | 0.1 | 0.1 | 0.1 |
| | Stearyl glycyrrhizinate | 0.1 | 0.1 | 0.1 | 0.1 | 0.1 | 0.1 | 0.1 | 0.1 | 0.1 |
| | Dimethicone | 2.4 | 2.4 | 2.4 | 2.4 | 2.4 | 2.4 | 2.4 | 2.4 | 2.4 |
| | Squalane | 4.8 | 4.8 | 4.8 | 4.8 | 4.8 | 4.8 | 4.8 | 4.8 | 4.8 |
| | Isononyl isononanoate | 2.4 | 2.4 | 2.4 | 2.4 | 2.4 | 2.4 | 2.4 | 2.4 | 2.4 |
| | Tocopherol | 0.2 | 0.2 | 0.2 | 0.2 | 0.2 | 0.2 | 0.2 | 0.2 | 0.2 |
| | Trioctanoin | 2.4 | 2.4 | 2.4 | 2.4 | 2.4 | 2.4 | 2.4 | 2.4 | 2.4 |
| | 2-Octyldodecanol | 2.4 | 2.4 | 2.4 | 2.4 | 2.4 | 2.4 | 2.4 | 2.4 | 2.4 |
| | Poly (10) glyceryl monostearate | 0.1 | 0.5 | 1.0 | 2.0 | 5.0 | 10.0 | 20.0 | | |
| | 1,3-Butylene glycol | 2.4 | 2.4 | 2.4 | 2.4 | 2.4 | 2.4 | 2.4 | 2.4 | 2.4 |
| | Glycerin | 4.8 | 4.8 | 4.8 | 4.8 | 4.8 | 4.8 | 4.8 | 4.8 | 4.8 |
| | Sodium hyaluronate (1% aqueous solution) | 4.8 | 4.8 | 4.8 | 4.8 | 4.8 | 4.8 | 4.8 | 4.8 | 4.8 |
| II | dl-α-Tocopherol dimethylglycinate | 1.0 | 1.0 | 1.0 | 1.0 | 1.0 | 1.0 | 1.0 | 1.0 | 1.0 |
| | Trisodium citrate | 0.4 | 0.4 | 0.4 | 0.4 | 0.4 | 0.4 | 0.4 | 0.4 | 0.4 |
| | Sodium chloride | 0.8 | 0.8 | 0.8 | 0.8 | 0.8 | 0.8 | 0.8 | 0.8 | 0.8 |
| | Potassium hydroxide | | | | | | | | | 0.1 |
| | Purified water | 64.9 | 64.5 | 64.0 | 63.0 | 60.0 | 55.0 | 45.0 | 67.4 | 64.9 |
| Storage stability test | | AA | AA | AA | AA | AA | AA | AA | BB | BB |
| Viscosity before test [mPa·s] | | 45000 | 45000 | 44000 | 45000 | 45000 | 45000 | 47000 | 22000 | 32000 |
| Viscosity after test [mPa·s] | | 45000 | 45000 | 45000 | 45000 | 45000 | 42000 | 47000 | 8000 | 10000 |

As shown in Table 7, Examples 43 to 49 used dextrin palmitate and poly (10) glyceryl monostearate, whilst Comparative Example 5 did not use dextrin palmitate and poly (10) glyceryl monostearate and Comparative Example 6 replaced dextrin palmitate and poly (10) glyceryl monostearate by palmitic acid and potassium hydroxide. The creams of Examples 43 to 49 were shown to have superior storage stability to the creams of Comparative Examples 5 and 6.

### [Example 50]

A cream was prepared as described above using the materials in the amounts shown in Table 8. The cream was tested for storage stability. The result is shown in Table 8, in which the values are % by mass except for the viscosity value.

**Table 8**

| | | Ex. |
|---|---|---|
| | | 50 |
| I | Glyceryl stearate | 2.0 |
| | Myristyl alcohol | 1.2 |
| | PEG-75 stearate | 1.2 |
| | Beeswax | 1.2 |
| | 1,2-Octanediol | 0.1 |
| | Diglyceryl PEG-9 | 3.0 |
| | Stearyl glycyrrhizinate | 0.1 |
| | Hydrogenated polyisobutene | 2.4 |
| | Hydrogenated polydecene | 4.8 |
| | Isopropyl isostearate | 4.8 |
| | Poly (10) glyceryl monoisostearate | 1.2 |
| | Liquid paraffin | 1.2 |
| | 2-Ethylhexyl palmitate | 2.4 |
| | Glycerin | 4.8 |
| | Dipropylene glycol | 0.4 |
| | Polysorbate 60 | 4.8 |
| | Isopentyl diol | 2.4 |
| | Acrylate/alkyl (C10-30) acrylate cross polymer | 0.2 |
| | dl-α-Tocopherol dimethylglycinate | 1.0 |
| II | Xanthan gum | 0.2 |
| | Polysaccharide from alcaligenes | 0.1 |
| | Hydrolyzed collagen | 0.1 |
| | Sodium hydroxide | 0.1 |
| | Maltitol | 0.1 |
| | Tetrasodium EDTA | 0.2 |
| | Purified water | 60.0 |
| Storage stability test | | AA |
| Viscosity before test [mPa·s] | | 52000 |
| Viscosity after test [mPa·s] | | 52000 |

As shown in Table 8, the cream of Example 50 was shown to have excellent storage stability.

### [Example 51]

A cream was prepared as described above using the materials in the amounts shown in Table 9. The cream was tested for storage stability. The result is shown in Table 9, in which the values are % by mass except for the viscosity value.

**Table 9**

| | | Ex. |
|---|---|---|
| | | 51 |
| I | Glyceryl stearate | 2.0 |
| | Cyclomethicone | 1.0 |
| | Squalane | 7.0 |
| | Isononyl isononanoate | 5.0 |
| | Glyceryl tri(caprylate/caprate) | 2.0 |
| | Cetyl palmitate | 1.0 |
| | Cholesterol | 0.5 |
| | Hydrogenated lecithin | 0.5 |
| | Poly (10) glyceryl myristate | 2.0 |
| | POE (60) hydrogenated castor oil | 0.4 |
| | Cetanol | 2.0 |
| | Stearyl alcohol | 1.0 |
| | Glyceryl tri-2-ethylhexanoate | 4.0 |
| | PEG-60 stearate | 1.0 |
| | Tocopherol acetate | 0.8 |
| | Phenoxyethanol | 0.4 |
| | Propylparaben | 0.1 |
| | dl-α-Tocopherol dimethylglycinate | 1.0 |
| II | Dipotassium glycyrrhizinate | 0.2 |
| | Carbomer | 0.1 |
| | Diglycerin | 3.0 |
| | Pentylene glycol | 2.0 |
| | Methylparaben | 0.1 |
| | Citric acid | 0.1 |
| | Trisodium citrate | 1.0 |
| | Tetrasodium EDTA | 0.2 |
| | Magnesium sulfate | 1.0 |
| | Potassium hydroxide | 0.1 |
| | Purified water | 60.5 |
| Storage stability test | | AA |
| Viscosity before test [mPa·s] | | 32000 |
| Viscosity after test [mPa·s] | | 32000 |

As shown in Table 9, the cream of Example 51 was shown to have excellent storage stability. with the proviso that preparations containing the following components (i) to (xii) are excluded:
(i) 0.20 w% of a tocopherol glycine ester, 3.00 wt% of a ascorbic acid-2-phosphoric acid ester, 72.10 wt% of purified water, 1.0 wt% of surfactin Na; or
   1.00 wt% of a tocopherol glycine ester, 9.00 wt% of a ascorbic acid-2-phosphoric acid ester, 65.30 wt% of purified water, 1.0 wt% of surfactin Na; or
   1.00 wt% of a tocopherol glycine ester, 1.00 wt% of a ascorbic acid-2-phosphoric acid ester, 73.30 wt% of purified water, 1.0 wt% of surfactin Na; or
   0.20.wt% of a tocopherol glycine ester, 3.00 wt% of a ascorbic acid-2-phosphoric acid ester, 73.00 wt% of purified water, 0.1 wt% of surfactin Na; or
   1.00 wt% of a tocopherol glycine ester, 1.00 wt% of a ascorbic acid-2-phosphoric acid ester, 74.20 wt% of purified water, 0.1 wt% of surfactin Na; or
   0.20.wt% of a tocopherol glycine ester, 3.00 wt% of a ascorbic acid-2-phosphoric acid ester, 73.10 wt% of purified water, 0.01 wt% of surfactin Na; or
   1.00 wt% of a tocopherol glycine ester, 1.00 wt% of a ascorbic acid-2-phosphoric acid ester, 74.30 wt% of purified water, 0.01 wt% of surfactin Na,
(ii) 7.00 wt% of 1,3-butylene glycol,
(iii) 0.10 wt% of xanthane gum,
(iv) 2.50 wt% of POE (20) sorbitan monostearate,
(v) 1.0 wt% of lipophilic glyceryl monostearate,
(vi) 0.50 wt% of stearic acid,
(vii) 1.50 wt% of behenyl alcohol,
(viii) 0.50 wt% of cetyl palmitate,
(ix) 5.00 wt% of squalane,
(x) 5.00 wt% of cetyl 2-ethylhexanoate,
(xi) 0.50 wt% of methyl polysiloxane (100 cSt), and
(xii) 0.10 wt% of methyl para-hydroxybenzoate,
wherein the tocopherol glycine ester is selected from the group consisting of dl-α-tocopherol dimethylglycine ester hydrochloride, d-α-tocopherol dimethylglycine ester hydrochloride, d-δ-tocopherol dimethylglycine ester hydrochloride, d-γ-tocopherol dimethylglycine ester hydrochloride, dl-α-tocopherol sarcosine ester hydrochloride, d-α-tocopherol sarcosine ester hydrochloride, d-δ-tocopherol sarcosine ester hydrochloride, and d-γ-tocopherol sarcosine ester hydrochloride,
and wherein the ascorbic acid-2-phosphoric acid ester is selected from the group consisting of ascorbic acid-2-phosphoric acid ester sodium salt, ascorbic acid-2-phosphoric acid ester magnesium salt, ascorbic acid-2-phosphoric acid ester-6-palmitic acid ester sodium salt and ascorbic acid-2-phosphoric acid ester-6-hexyldecanoic acid ester sodium salt.

## Claims

1. An emulsified skin external preparation comprising 0.03 to 25% by mass of a tocopherol glycinate derivative, and 0.05 to 25% by mass of at least one fatty acid ester selected from the group consisting of glycerin mono fatty acid ester, polyglycerin fatty acid ester and dextrin fatty acid ester, the tocopherol glycinate derivative being represented by Formula (1): 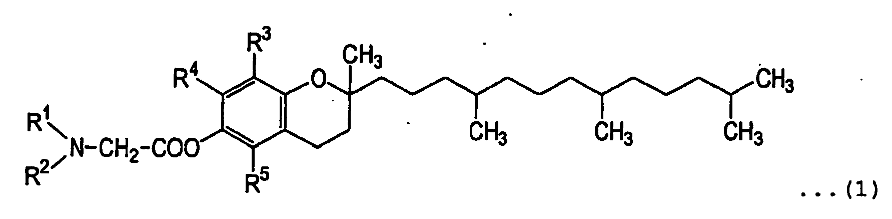 wherein R¹ and R² are each a lower alkyl group or a hydrogen atom and cannot be hydrogen atoms at the same time, and R³, R⁴ and R⁵ are each a hydrogen atom or a methyl group,
0.20.wt% of a tocopherol glycine ester, 3.00 wt% of a ascorbic acid-2-phosphoric acid ester, 73.10 wt% of purified water, 0.01 wt% of surfactin Na; or
1.00 wt% of a tocopherol glycine ester, 1.00 wt% of a ascorbic acid-2-phosphoric acid ester, 74.30 wt% of purified water, 0.01 wt% of surfactin Na, wherein the tocopherol glycine ester is selected from the group consisting of dl-α-tocopherol dimethylglycine ester hydrochloride, d-α-tocopherol dimethylglycine ester hydrochloride, d-δ-tocopherol dimethylglycine ester hydrochloride, d-γ-tocopherol dimethylglycine ester hydrochloride, dl-α-tocopherol sarcosine ester hydrochloride, d-α-tocopherol sarcosine ester hydrochloride, d-δ-tocopherol sarcosine ester hydrochloride, and d-γ-tocopherol sarcosine ester hydrochloride,
and wherein the ascorbic acid-2-phosphoric acid ester is selected from the group consisting of ascorbic acid-2-phosphoric acid ester sodium salt, ascorbic acid-2-phosphoric acid ester magnesium salt, ascorbic acid-2-phosphoric acid ester-6-palmitic acid ester sodium salt and ascorbic acid-2-phosphoric acid ester-6-hexyldecanoic acid ester sodium salt.

2. The emulsified skin external preparation according to claim 1, wherein the tocopherol glycinate derivative is tocopherol N,N-dimethylglycinate or tocopherol sarcosinate.

3. The emulsified skin external preparation according to claim 1 or 2, wherein the tocopherol glycinate with the proviso that preparations containing the following components (i) to (xii) are excluded:
(i) 0.20 w% of a tocopherol glycine ester, 3.00 wt% of a ascorbic acid-2-phosphoric acid ester, 72.10 wt% of purified water, 1.0 wt% of surfactin Na; or
1.00 wt% of a tocopherol glycine ester, 9.00 wt% of a ascorbic acid-2-phosphoric acid ester, 65.30 wt% of purified water, 1.0 wt% of surfactin Na; or
1.00 wt% of a tocopherol glycine ester, 1.00 wt% of a ascorbic acid-2-phosphoric acid ester, 73.30 wt% of purified water, 1.0 wt% of surfactin Na; or
0.20 wt% of a tocopherol glycine ester, 3.00 wt% of a ascorbic acid-2-phosphoric acid ester, 73.00 wt% of purified water, 0.1 wt% of surfactin Na; or
1.00 wt% of a tocopherol glycine ester, 1.00 wt% of a ascorbic acid-2-phosphoric acid ester, 74.20 wt% of purified water, 0.1 wt% of surfactin Na; or
0.20.wt% of a tocopherol glycine ester, 3.00 wt% of a ascorbic acid-2-phosphoric acid ester, 73.10 wt% of purified water, 0.01 wt% of surfactin Na; or
1.00 wt% of a tocopherol glycine ester, 1.00 wt% of a ascorbic acid-2-phosphoric acid ester, 74.30 wt% of purified water, 0.01 wt% of surfactin Na.
(ii) 7.00 wt% of 1,3-butylene glycol,
(iii) 0.10 wt% of xanthane gum,
(iv) 2.50 wt% of POE (20) sorbitan monostearate,
(v) 1.0 wt% of lipophilic glyceryl monostearate,
(vi) 0.50 wt% of stearic acid,
(vii) 1.50 wt% of behenyl alcohol,
(viii) 0.50 wt% of cetyl palmitate,
(ix) 5.00 wt% of squalane,
(x) 5.00 wt% of cetyl 2-ethylhexanoate,
(xi) 0.50 wt% of methyl polysiloxane (100 cSt), and
(xii) 0.10 wt% of methyl para-hydroxybenzoate,
wherein the tocopherol glycine ester is selected from the group consisting of dl-α-tocopherol dimethylglycine ester hydrochloride, d-α-tocopherol dimethylglycine ester hydrochloride, d-δ-tocopherol dimethylglycine ester hydrochloride, d-γ-tocopherol dimethylglycine ester hydrochloride, dl-α-tocopherol sarcosine ester hydrochloride, d-α-tocopherol sarcosine ester hydrochloride, d-δ-tocopherol sarcosine ester hydrochloride, and d-γ-tocopherol sarcosine ester hydrochloride,
and wherein the ascorbic acid-2-phosphoric acid ester is selected from the group consisting of ascorbic acid-2-phosphoric acid ester sodium salt, ascorbic acid-2-phosphoric acid ester magnesium salt, ascorbic acid-2-phosphoric acid ester-6-palmitic acid ester sodium salt and ascorbic acid-2-phosphoric acid ester-6-hexyldecanoic acid ester sodium salt.
derivative is at least one derivative selected from the group consisting of α-tocopherol glycinate derivatives, γ-tocopherol glycinate derivatives and δ-tocopherol glycinate derivatives.

4. The emulsified skin external preparation according to any one of claims 1 to 3, wherein the tocopherol glycinate derivative is at least one derivative selected from the group consisting of dl-α-tocopherol glycinate derivatives, d-α-tocopherol glycinate derivatives, d-γ-tocopherol glycinate derivatives and d-δ-tocopherol glycinate derivatives.

5. The emulsified skin external preparation according to claim 1, wherein the glycerin mono fatty acid ester is at least one fatty acid ester selected from the group consisting of glyceryl laurate, glyceryl myristate, glyceryl stearate, glyceryl isostearate, glyceryl behenate, glyceryl linoleate, glyceryl oleate, glyceryl cocofatty acid ester, glyceryl ricinoleate, glyceryl hydroxystearate and glyceryl erucate.

6. The emulsified skin external preparation according to claim 1, wherein the polyglycerin fatty acid ester
is a fatty acid ester of polyglycerin having two to ten glycerin molecules.

7. The emulsified skin external preparation according to claim 6, wherein the fatty acid ester of polyglycerin having two to ten glycerin molecules is at least one fatty acid ester selected from the group consisting of poly (2) glyceryl monolaurate, poly (2) glyceryl monomyristate, poly (2) glyceryl monostearate, poly (2) glyceryl monoisostearate, poly (10) glyceryl monolaurate, poly (10) glyceryl monomyristate, poly (10) glyceryl monostearate and poly (10) glyceryl monoisostearate.

8. The emulsified skin external preparation according to claim 1, wherein the dextrin fatty acid ester is at least one fatty acid ester selected from the group consisting of dextrin myristate, dextrin palmitate, dextrin (palmitate/octanoate) and dextrin stearate.

9. An emulsified cosmetic comprising the emulsified skin external preparation claimed in any one of claims 1 to 8.

10. A method for stabilizing an emulsified skin external preparation,
**characterized in that** a tocopherol glycinate derivative is used in combination with at least one fatty acid ester selected from the group consisting of glycerin mono fatty acid ester, polyglycerin fatty acid ester and dextrin fatty acid ester, the tocopherol glycinate derivative being represented by Formula (1): 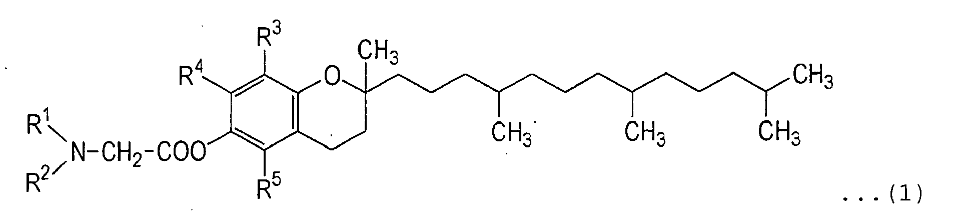 wherein R¹ and R² are each a lower alkyl group or a hydrogen atom and cannot be hydrogen atoms at the same time, and R³, R⁴ and R⁵ are each a hydrogen atom or a methyl group,
with the proviso that the claimed method does not apply to the preparations excluded by the disclaimer of claim 1.

## Patentansprüche

1. Emulgierte topische Hautzubereitung, das 0,03 bis 25 Massen-% eines Tocopherolglycinatderivats und 0,05 bis 25 Massen-% mindestens eines Fettsäureesters enthält, der aus der Gruppe ausgewählt ist, die aus Glycerinmonofettsäureester, Polyglycerinfettsäureester und Dextrinfettsäureester besteht, wobei das Tocopherolglycinatderivat durch die Formel (1) dargestellt ist: 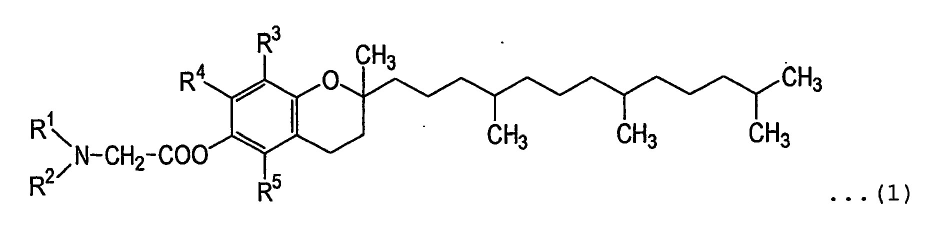 worin R¹ und R² jeweils eine Niederalkylgruppe oder ein Wasserstoffatom sind und nicht gleichzeitig Wasserstoffatome sein können, und R³, R⁴ und R⁵ jeweils ein Wasserstoffatom oder eine Methylgruppe sind,
mit der Maßgabe, dass die Zubereitungen ausgeschlossen sind, die die folgenden Komponenten (i) bis (xii) enthalten:
(i) 0,20 Gew.-% eines Tocopherolglycinesters, 3,00 Gew.-% eines Ascorbinsäure-2-phosphorsäureesters, 72,10 Gew.-% gereinigtes Wasser, 1,0 Gew.-% Natriumsurfactin; oder
1,00 Gew.-% eines Tocopherolglycinesters, 9,00 Gew.-% eines Ascorbinsäure-2-phosphorsäureesters, 65,30 Gew.-% gereinigtes Wasser, 1,0 Gew.-% Natriumsurfactin; oder
1,00 Gew.-% eines Tocopherolglycinesters, 1,00 Gew.-% eines Ascorbinsäure-2-phosphorsäureesters, 73,30 Gew.-% gereinigtes Wasser, 1,0 Gew.-% Natriumsurfactin; oder
0,20 Gew.-% eines Tocopherolglycinesters, 3,00 Gew.-% eines Ascorbinsäure-2-phosphorsäureesters, 73,00 Gew.-% gereinigtes Wasser, 0,1 Gew.-% Natriumsurfactin; oder
1,0 Gew.-% eines Tocopherolglycinesters, 1,00 Gew.-% eines Ascorbinsäure-2-phosphorsäureesters, 74,20 Gew.-% gereinigtes Wasser, 0,1 Gew.-% Natriumsurfactin; oder
0,2 Gew.-% eines Tocopherolglycinesters, 3,00 Gew.-% eines Ascorbinsäure-2-phosphorsäureesters, 73,10 Gew.-% gereinigtes Wasser, 0,01 Gew.-% Natriumsurfactin; oder
1,0 Gew.-% eines Tocopherolglycinesters, 1,00 Gew.-% eines Ascorbinsäure-2-phosphorsäureesters, 74,30 Gew.-% gereinigtes Wasser, 0,01 Gew.-% Natriumsurfactin.
(ii) 7,0 Gew.-% 1,3-Butylenglycol,
(iii) 0,10 Gew.-% Xanthangummi,
(iv) 2,50 Gew.-% POE (20) Sorbitanmonostearat,
(v) 1,0 Gew.-% lipophiles Glycerylmonostearat,
(vi) 0,50 Gew.-% Stearinsäure,
(vii) 1,50 Gew.-% Behenalkohol,
(viii) 0,50 Gew.-% Cetylpalmitat,
(ix) 5,00 Gew.-% Squalan,
(x) 5,00 Gew.-% Cetyl-2-ethylhexanoat,
(xi) 0,50 Gew.-% Methylpolysiloxan (100 cSt), und
(xii) 0,10 Gew.-% Methyl-para-hydroxybenzoat,
wobei der Tocopherolglycinester aus der Gruppe ausgewählt ist, die aus dl-α-Tocopheroldimethylglycinester-Hydrochlorid, d-α-Tocopheroldimethylglycinester-Hydrochlorid, d-δ-Tocopheroldimethylglycinester-Hydrochlorid, d-γ-Tocopheroldimethylglycinester-Hydrochlorid, dl-α-Tocopherolsarcosinester-Hydrochlorid, d-α-Tocopherolsarcosinester-Hydrochlorid, d-δ-Tocophoerolsarcosinester-Hydrochlorid und d-γ-Tocopherolsarcosinester-Hydrochlorid besteht,
und wobei der Ascorbinsäure-2-phosphorsäureester aus der Gruppe ausgewählt ist, die aus Ascorbinsäure-2-phosphorsäureester-Natriumsalz, Ascorbinsäure-2-phosphorsäureester-Magnesiumsalz, Ascorbinsäure-2-phosphorsäureester-6-palmitinsäureester-Natriumsalz und Ascorbinsäure-2-phosphorsäureester-6-hexyldecansäureester-Natriumsalz besteht.

2. Emulgierte topische Hautzubereitung nach Anspruch 1, wobei das Tocopherolglycinatderivat Tocopherol-N,N-dimethylglycinat oder Tocopherolsarcosinat ist.

3. Emulgierte topische Hautzubereitung nach Anspruch 1 oder 2, wobei das Tocopherolglycinatderivat mindestens ein Derivat ist, das aus der Gruppe ausgewählt ist, die aus α-Tocopherolglycinatderivaten, γ-Tocopherolglycinatderivaten und δ-Tocopherolglycinatderivaten besteht.

4. Emulgierte topische Hautzubereitung nach einem der Ansprüche 1 bis 3, wobei das Tocopherolglycinatderivat mindestens ein Derivat ist, das aus der Gruppe ausgewählt ist, die aus dl-α-Tocopherolglycinatderivaten, d-α-Tocopherolglycinatderivaten, d-γ-Tocopherolglycinatderivaten und d-δ-Tocopherolglycinatderivaten besteht.

5. Emulgierte topische Hautzubereitung nach Anspruch 1, wobei der Glycerinmonofettsäureester mindestens ein Fettsäureester ist, der aus der Gruppe ausgewählt ist, die aus Glyceryllaurat, Glycerylmyristat, Glycerylstearat, Glycerylisostearat, Glycerlybehenat, Glyceryllinoleat, Glyceryloleat, Glyceryl-Kokosnussfettsäureester, Glycerylricinoleat, Glycerylhydroxystearat und Glycerylerucat besteht.

6. Emulgierte topische Hautzubereitung nach Anspruch 1, wobei der Polyglycerinfettsäureester ein Fettsäureester aus Polyglycerin mit zwei bis zehn Glycerinmolekülen ist.

7. Emulgierte topische Hautzubereitung nach Anspruch 6, wobei der Fettsäureester aus Polyglycerin mit zwei bis zehn Glycerinmolekülen mindestens ein Fettsäureester ist, der aus der Gruppe ausgewählt ist, die aus Poly(2)glycerylmonolaurat, Poly(2)glycerylmonomyristat, Poly(2)glycerylmonostearat, Poly(2)glycerylmonoisostearat, Poly(10)glycerylmonolaurat, Poly(10)glycerylmonomyristat, Poly(10)glycerylmonostearat und Poly(10)glycerylmonoisostearat besteht.

8. Emulgierte topische Hautzubereitung nach Anspruch 1, wobei der Dextrinfettsäureester mindestens ein Fettsäureester ist, der aus der Gruppe ausgewählt ist, die aus Dextrinmyristat, Dextrinpalmitat, Dextrin(palmitat/octanoat) und Dextrinstearat besteht.

9. Emulgiertes Kosmetikum, welches die emulgierte topische Hautzubereitung nach einem der Ansprüche 1 bis 8 enthält.

10. Verfahren zum Stabilisieren einer emulgierten topischen Hautzubereitung, **dadurch gekennzeichnet, dass** ein Tocopherolglycinatderivat in Kombination mit mindestens einem Fettsäureester eingesetzt wird, der aus der Gruppe ausgewählt ist, die aus Glycerinmonofettsäureester, Polyglycerinfettsäureester und Dextrinfettsäureester besteht, wobei das Tocopherolglycinatderivat durch die Formel (1) dargestellt ist: 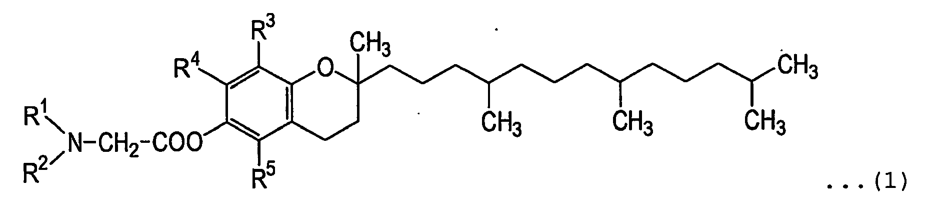 worin R¹ und R² jeweils eine Niederalkylgruppe oder ein Wasserstoffatom sind und nicht gleichzeitig Wasserstoffatome sein können, und R³, R⁴ und R⁵ jeweils ein Wasserstoffatom oder eine Methylgruppe darstellen,
mit der Maßgabe, dass das beanspruchte Verfahren nicht auf die Zubereitungen zutrifft, die durch die Ausschlusserklärung nach Anspruch 1 ausgenommen sind.

## Revendications

1. Préparation externe cutanée émulsifiée comprenant 0,03 à 25 % en masse d'un dérivé de glycinate de tocophérol et 0,05 à 25 % en masse d'au moins un ester d'acide gras choisi dans le groupe consistant en un monoester de glycérine et d'acide gras, un ester de polyglycérine et d'acide gras et un ester de dextrine et d'acide gras, le dérivé de glycinate de tocophérol étant représenté par la formule (1) : 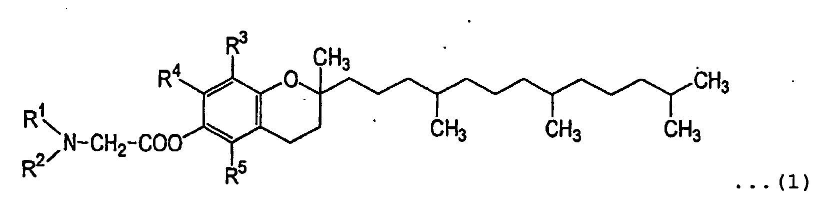 où R¹ et R² sont chacun un groupe alkyle inférieur ou un atome d'hydrogène et ne peuvent pas être des atomes d'hydrogène en même temps, et R³, R⁴ et R⁵ sont chacun un atome d'hydrogène ou un groupe méthyle, avec la condition que les préparations contenant les composants (i) à (xii) suivants soient exclues :
(i) 0,20 % en poids d'un ester de tocophérol et de glycine, 3,00 % en poids d'un 2-phosphate d'acide ascorbique, 72,10 % en poids d'eau purifiée, 1,0 % en poids de surfactine Na ; ou
1,00 % en poids d'un ester de tocophérol et de glycine, 9,00 % en poids d'un 2-phosphate d'acide ascorbique, 65,30 % en poids d'eau purifiée, 1,0 % en poids de surfactine Na ; ou
1,00 % en poids d'un ester de tocophérol et de glycine, 1,00 % en poids d'un 2-phosphate d'acide ascorbique, 73,30 % en poids d'eau purifiée, 1,0 % en poids de surfactine Na ; ou
0,20 % en poids d'un ester de tocophérol et de glycine, 3,00 % en poids d'un 2-phosphate d'acide ascorbique, 73,00 % en poids d'eau purifiée, 0,1 % en poids de surfactine Na ; ou
1,00 % en poids d'un ester de tocophérol et de glycine, 1,00 % en poids d'un 2-phosphate d'acide ascorbique, 74,20 % en poids d'eau purifiée, 0,1 % en poids de surfactine Na ; ou
0,20 % en poids d'un ester de tocophérol et de glycine, 3,00 % en poids d'un 2-phosphate d'acide ascorbique, 73,10 % en poids d'eau purifiée, 0,01 % en poids de surfactine Na ; ou
1,00 % en poids d'un ester de tocophérol et de glycine, 1,00 % en poids d'un 2-phosphate d'acide ascorbique, 74,30 % en poids d'eau purifiée, 0,01 % en poids de surfactine Na,
(ii) 7,00 % en poids de 1,3-butylèneglycol,
(iii) 0,10 % en poids de gomme de xanthane,
(iv) 2,50 % en poids de monostéarate POE (20) de sorbitan,
(v) 1,0 % en poids de monostéarate de glycéryle lipophile,
(vi) 0,50 % en poids d'acide stéarique,
(vii) 1,50 % en poids d'alcool béhénylique,
(viii) 0,50 % en poids de palmitate de cétyle,
(ix) 5,00 % en poids de squalane,
(x) 5,00 % en poids de 2-éthylhexanoate de cétyle,
(xi) 0,50 % en poids de méthylpolysiloxane (100 cSt), et
(xii) 0,10 % en poids de para-hydroxybenzoate de méthyle,
où l'ester de tocophérol et de glycine est choisi dans le groupe consistant en le chlorhydrate d'ester de dl-α-tocophérol et de diméthylglycine, le chlorhydrate d'ester de d-α-tocophérol et de diméthylglycine, le chlorhydrate d'ester de d-δ-tocophérol et de diméthylglycine, le chlorhydrate d'ester de d-γ-tocophérol et de diméthylglycine, le chlorhydrate d'ester de dl-α-tocophérol et de sarcosine, le chlorhydrate d'ester de d-α-tocophérol et de sarcosine, le chlorhydrate d'ester de d-δ-tocophérol et de sarcosine et le chlorhydrate d'ester de d-γ-tocophérol et de sarcosine,
et où le 2-phosphate d'acide ascorbique est choisi dans le groupe consistant en le sel de sodium de 2-phosphate d'acide ascorbique, le sel de magnésium de 2-phosphate d'acide ascorbique, le sel de sodium de 2-phosphate-6-palmitate d'acide ascorbique et le sel de sodium de 2-phosphate-6-hexyldécanoate d'acide ascorbique.

2. Préparation externe cutanée émulsifiée selon la revendication 1, où le dérivé de glycinate de tocophérol est le N,N-diméthylglycinate de tocophérol ou le sarcosinate de tocophérol.

3. Préparation externe cutanée émulsifiée selon la revendication 1 ou 2, où le dérivé de glycinate de tocophérol est au moins un dérivé choisi dans le groupe consistant en les dérivés de glycinate d'α-tocophérol, les dérivés de glycinate de γ-tocophérol et les dérivés de glycinate de δ-tocophérol.

4. Préparation externe cutanée émulsifiée selon l'une quelconque des revendications 1 à 3, où le dérivé de glycinate de tocophérol est au moins un dérivé choisi dans le groupe consistant en les dérivés de glycinate de dl-α-tocophérol, les dérivés de glycinate de d-α-tocophérol, les dérivés de glycinate de d-γ-tocophérol et les dérivés de glycinate de d-δ-tocophérol.

5. Préparation externe cutanée émulsifiée selon la revendication 1, où le monoester d'acide gras et de glycérine est au moins un ester d'acide gras choisi dans le groupe consistant en le laurate de glycéryle, le myristate de glycéryle, le stéarate de glycéryle, l'isostéarate de glycéryle, le béhénate de glycéryle, le linoléate de glycéryle, l'oléate de glycéryle, un ester d'acide gras de copra et de glycéryle, le ricinoléate de glycéryle, l'hydroxystéarate de glycéryle et l'érucate de glycéryle.

6. Préparation externe cutanée émulsifiée selon la revendication 1, où l'ester d'acide gras et de polyglycérine est un ester d'acide gras et de polyglycérine ayant deux à dix molécules de glycérine.

7. Préparation externe cutanée émulsifiée selon la revendication 6, où l'ester d'acide gras et de polyglycérine ayant deux à dix molécules de glycérine est au moins un ester d'acide gras choisi dans le groupe consistant en le poly (2) monolaurate de glycéryle, le poly (2) monomyristate de glycéryle, le poly (2) monostéarate de glycéryle, le poly (2) monoisostéarate de glycéryle, le poly (10) monolaurate de glycéryle, le poly (10) monomyristate de glycéryle, le poly (10) monostéarate de glycéryle et le poly (10) monoisostéarate de glycéryle.

8. Préparation externe cutanée émulsifiée selon la revendication 1, où l'ester d'acide gras et de dextrine est au moins un ester d'acide gras choisi dans le groupe consistant en le myristate de dextrine, le palmitate de dextrine, le (palmitate/octanoate) de dextrine et le stéarate de dextrine.

9. Cosmétique émulsifié comprenant la préparation externe cutanée émulsifiée selon l'une quelconque des revendications 1 à 8.

10. Procédé pour stabiliser une préparation externe cutanée émulsifiée **caractérisé en ce qu'**un dérivé de glycinate de tocophérol est utilisé en combinaison avec au moins un ester d'acide gras choisi dans le groupe consistant en un monoester d'acide gras et de glycérine, un ester d'acide gras et de polyglycérine et un ester d'acide gras et de dextrine, le dérivé de glycinate de tocophérol étant représenté par la formule (1) : 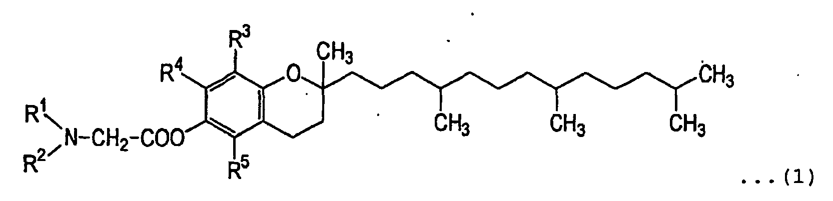 où R¹ et R² sont chacun un groupe alkyle inférieur ou un atome d'hydrogène et ne peuvent pas être des atomes d'hydrogène en même temps, et R³, R⁴ et R⁵ sont chacun un atome d'hydrogène ou un groupe méthyle, avec la condition que le procédé revendiqué ne s'applique pas aux préparations exclues par l'exclusion selon la revendication 1.
